# EUROPEAN PATENT APPLICATION

(11) **EP 4 060 047 A1**
(43) Date of publication of application: **21.09.2022**
(21) Application number: 20888262.1
(22) Date of filing: 13.11.2020
(51) Int. Cl.: C12Q 1/68, C12Q 1/6837, C12Q 1/6876

(54) **METHOD FOR DETECTING OR QUANTIFYING OLIGONUCLEOTIDE**

(30) Priority: 14.11.2019 JP 2019206380
(71) Applicant: Sekisui Medical Co., Ltd., Tokyo 103-0027 (JP)
(72) Inventor: OSAWA, Masako, Tokyo 103-0027 (JP); OE, Aya, Tokyo 103-0027 (JP); OGAWA, Funa, Tokyo 103-0027 (JP)
(74) Representative: Hoffmann Eitle
(86) International application number: PCT/JP2020/042363
(87) International publication number: WO 2021/095829

(57) **Abstract**

An object of the present invention is to increase the reactivity of poly A polymerase with an oligonucleotide or a DNA in which the nucleic acid base at the 3'-end is chemically modified, and then to improve the sensitivity of detection through a PALSAR method or the like. The present invention relates to increasing the reactivity of poly A polymerase with an oligonucleotide or a DNA used for the nucleic acid medicine, in which the nucleic acid base at the 3'-end is chemically modified, and then adjusting the Mn²⁺ concentration during a poly A polymerase reaction, in a method of detecting the oligonucleotide, for a detection target that is an oligonucleotide or a DNA in which the nucleic acid base at the 3'-end is chemically modified, through steps of addition of poly A to the 3'-end of the oligonucleotide with poly A polymerase, capturing with a capture probe, and amplification by a PALSAR method or the like. Thus, the problem of the present invention is solved.

SEQUENCE LISTING

<110>  SEKISUI MEDICAL CO., LTD.

<120>  Method for detecting or quantifying oligonucleotide

<130>  20P00785

<160>  4

<170>  PatentIn version 3.5

<210>  1
<211>  16
<212>  DNA
<213>  Artificial Sequence

<220>
<223>  target-1

<400>  1
tgagctgact tgatgc                                                        16

<210>  2

<211>  16

<212>  DNA

<213>  Artificial Sequence

<220>

<223>  CP-1

<400>  2

gcatcaagtc agctca                                                       16

<210>  3

<211>  46

<212>  DNA

<213>  Artificial Sequence

<220>

<223>  HCP-1

<400>  3

caacaatcag gacgataccg atgaagtttt tttttttttt tttttt                      46

<210>  4

<211>  46

<212>  DNA

<213>  Artificial Sequence

<220>

<223>  HCP-2

<400>  4

gtcctgattg ttgcttcatc ggtatcaaaa aaaaaaaaaa aaaaaa                    46

SEQUENCE LISTING

<110>  SEKISUI MEDICAL Co., Ltd.

<120>  Detection method of oligonucleotide

<130>  19P01287

<150>  JP 2019-206380
<151>  2019-11-14

<160>  5

<170>  PatentIn version 3.5

<210>  1
<211>  16
<212>  DNA
<213>  Artificial Sequence

<220>
<223>  target-1

<400>  1
tgagctgact tgatgc
\201@\201@\201@ 16


<210>  2
<211>  16
<212>  DNA
<213>  Artificial Sequence

<220>
<223>  CP-1

<400>  2
gcatcaagtc agctca
\201@\201@16


<210>  3
<211>  46
<212>  DNA
<213>  Artificial Sequence

<220>
<223>  HCP-1

<400>  3
caacaatcag gacgataccg atgaagtttt tttttttttt tttttt                    46


<210>  4
<211>  46
<212>  DNA
<213>  Artificial Sequence

<220>
<223>  HCP-2

<400>  4
gtcctgattg ttgcttcatc ggtatcaaaa aaaaaaaaaa aaaaaa                    46

<210> 5
<211> 16
<212> DNA
<213> Artificial Sequence

<220>
<223> target-2

<400> 5
tgagctgact tgatgt
\201@\201@\201@ 16

## Description

### TECHNICAL FIELD

The present invention relates to a method for detecting or quantifying an oligonucleotide, which has high sensitivity and excellent quantitativeness, particularly relates to a high sensitivity detection or quantification method of an oligonucleotide and a DNA in which the nucleic acid base at the 3'-end is chemically modified.

### BACKGROUND ART

APALSAR method is known as a technique of amplifying and detecting oligonucleotides such as a nucleic acid or a DNA. The principle of the PALSAR method is to use one set of previously prepared DNA probes composed of three complementary regions (in the present specification, also called self-aggregating probes), in which the probes are labelled, and these form a mesh-like large DNA lump by repeating a self-aggregating reaction through hybridization. Thus, this is used as an amplification signal. In this method, this large DNA lump is bound to a DNA or an RNA as a detection target, so that the amplified signal is captured to detect and quantify the DNA or the RNA as the detection target.

A method of detecting a detection target such as an microRNA through a PALSAR method includes the following steps. This method includes: firstly, a step of adding poly A to the 3'-end of the microRNA with a poly A polymerase; next, a step of causing a capture probe to react with the microRNA to which the poly A is added to capture the microRNA; a step of causing the microRNA captured by the capture probe to react with a self-aggregating probe, so as to form a detection complex including the microRNA, the capture probe, and a probe polymer; and a detection step (Patent Document 1).

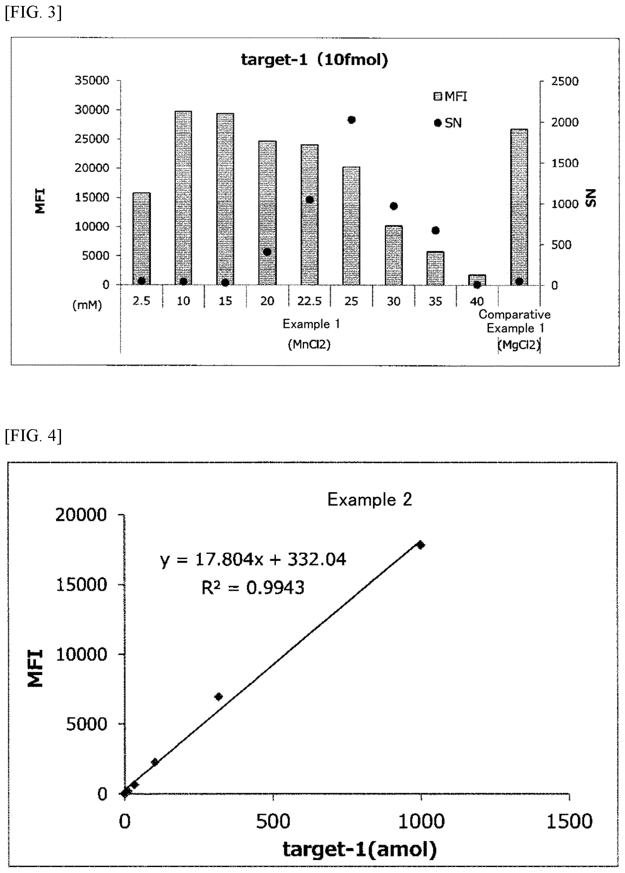

Here, it is known that the poly A polymerase generally requires Mg²⁺ or Mn²⁺, and in Non-Patent Document 1, the graph illustrates the uptake amount of AMP in a primer RNA (E. coli K12 strain tRNA) when Mg²⁺ or Mn²⁺ is added at each concentration (Fig. 3).

According to this drawing, it is illustrated that the poly A polymerase shows no activity in the absence of Mg²⁺ or Mn²⁺, and shows the maximum activity in the case where Mn²⁺ is 2.5 mM, and Mg²⁺ is 10 mM. Furthermore, this discloses that when both metal ions are added together at concentrations by which maximum activities are exhibited, respectively (Mn²⁺ 2.5 mM and Mg²⁺ 10 mM), the highest activity of the poly A polymerase is further obtained (Fig. 3B).

### CITATION LIST

### PATENT LITERATURE

Patent Document 1: Pamphlet No. WO2013-172305

### NON PATENT LITERATURE

Non-Patent Document 1: Eur. J. Biochem. 37, 31-40 (1973)

### SUMMARY OF INVENTION

### TECHNICAL PROBLEM

Here, we made an attempt to detect an oligonucleotide, for a detection target that is the oligonucleotide or the DNAused for the nucleic acid medicine, in which the nucleic acid base at the 3'-end is chemically modified, through steps of addition of poly A to the 3'-end with poly A polymerase, capturing by a capture probe, and amplification by a PALSAR method. As a result, we have found a new problem that in the case where the addition was carried out at the Mg²⁺ concentration of the above concentration in the reaction step of the poly A polymerase, the object to be detected cannot be measured due to a low signal and a high background value at the time of detection.

Therefore, an object of the present invention is to enhance the reactivity of the poly A polymerase with the oligonucleotide or the DNA in which the nucleic acid base at the 3'-end is chemically modified, and then to improve the sensitivity of the detection by the PALSAR method. In particular, when an attempt is made to detect the oligonucleotide in a biological sample such as blood plasma or brain, the detection sensitivity is hardly obtained under the conventional reaction conditions for poly A polymerase. Thus, increasing the detection sensitivity in the biological sample such as blood plasma or brain is a more difficult task.

### SOLUTION TO PROBLEM

The present invention is to increase the reactivity of poly A polymerase with an oligonucleotide or a DNA used for the nucleic acid medicine, in which the nucleic acid base at the 3'-end is chemically modified. Further, in a method of detecting the oligonucleotide, for a detection target that is an oligonucleotide or a DNA in which the nucleic acid base at the 3'-end is chemically modified, through steps of addition of poly A to the 3'-end of the oligonucleotide with poly A polymerase, capturing with a capture probe, and amplification by a PALSAR method or the like, it was found that by adjusting the Mn²⁺ concentration during a poly A polymerase reaction, it is possible to increase the signal and suppress the background, and to greatly increase the signal-to-noise ratio (SN ratio), thereby further increasing the detection sensitivity. Then, the present invention has been completed. That is, the present invention has the following configuration.
(1) A method of adding poly A to a 3'-end of a target oligonucleotide in a sample, in which the target oligonucleotide is an oligonucleotide or a DNA in which a nucleic acid base at the 3'-end is chemically modified, the method including:
   (i) bringing poly A polymerase into contact with the target oligonucleotide in the sample in the presence of Mn²⁺.
(2) A method of collecting a target oligonucleotide in a sample, in which the target oligonucleotide is an oligonucleotide or a DNA in which a nucleic acid base at a 3'-end is chemically modified, the method including:
   (i) bringing poly A polymerase into contact with the target oligonucleotide in the sample in the presence of Mn²⁺, thereby adding poly A to the 3'-end of the target oligonucleotide;
   (ii) bringing the sample into contact with a capture probe for capturing the target oligonucleotide to perform hybridization;
      in which the capture probe includes
      (A) a nucleic acid probe, and
      (B) a solid phase, an adapter, or a linker adjacent to a nucleotide at a 3'-end or 5'-end of the nucleic acid probe, and
         the nucleic acid probe contains an entire sequence or a partial sequence of the target oligonucleotide; and
   (iii) collecting a hybridization product included in the sample.
(3) A method of detecting a target oligonucleotide in a sample, in which the target oligonucleotide is an oligonucleotide or a DNA in which a nucleic acid base at a 3'-end is chemically modified, the method including:
   (i) bringing poly A polymerase into contact with the target oligonucleotide in the sample in the presence of Mn²⁺, thereby adding poly A to the 3'-end of the target oligonucleotide;
   (ii) bringing the sample into contact with a capture probe for capturing the target oligonucleotide to perform hybridization;
      in which the capture probe includes
      (A) a nucleic acid probe, and
      (B) a solid phase, an adapter, or a linker adjacent to a nucleotide at a 3'-end or 5'-end of the nucleic acid probe, and
         the nucleic acid probe contains an entire sequence or a partial sequence of the target oligonucleotide;
   (iii) collecting a hybridization product included in the sample; and
   (iv) detecting the collected hybridization product.
(4) A method of detecting a target oligonucleotide in a sample, in which the target oligonucleotide is an oligonucleotide or a DNA in which a nucleic acid base at a 3'-end is chemically modified, the method including the following steps:
   (i) bringing poly A polymerase into contact with the target oligonucleotide in the sample in the presence of Mn²⁺, thereby adding poly A to the 3'-end of the target oligonucleotide;
   (ii) bringing the sample into contact with a capture probe for capturing the target oligonucleotide to perform hybridization;
      in which the capture probe includes
      (A) a nucleic acid probe, and
      (B) a solid phase, an adapter, or a linker adjacent to a nucleotide at a 3'-end or 5'-end of the nucleic acid probe, and
         the nucleic acid probe contains an entire sequence or a partial sequence of the target oligonucleotide;
   (iii) bringing a hybridization product included in the sample, into contact with a pair of self-aggregating signal amplification probes composed of first and second oligonucleotides, thereby forming a complex of the hybridization product and an oligonucleotide polymer obtained through self-aggregation of the first and second oligonucleotides; and
   (iv) detecting the complex.
(5) The detection method described in (4), in which at least one of the first and second oligonucleotides contains a poly T sequence.
(6) The detection method described in (4) or (5), in which the step (iv) of forming the complex includes the step of bringing into contact with an assist probe,
   in which the assist probe contains
   a poly T sequence, and a sequence complementary to an entire sequence or a partial sequence of at least one of the first and second oligonucleotides.
(7) The detection method described in any one of (4) to (6), in which the first oligonucleotide contains at least a nucleic acid region X, a nucleic acid region Y, and a nucleic acid region Z or a nucleic acid region Z containing a poly T sequence in order from an 5'-end side, and
   the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.
(8) The method described in any one of (1) to (7), in which the sample is a blood-derived component, a brain-derived component, a buffer, or water, which contains the target oligonucleotide.
(9) The method described in any one of (1) to (8), in which the oligonucleotide in which the nucleic acid base at the 3'-end is chemically modified is LNA, BNA, phosphorothioate, morpholino oligo, boranophosphate, 2'-O-methylated RNA(2'-OMe), 2'-O-methoxyethylated RNA(2'-MOE), or 2'-F.
(10) The method described in (9), in which the oligonucleotide in which the nucleic acid base at the 3'-end is chemically modified is LNA, BNA, MOE, or OMe.
(11) The method described in any one of (1) to (10), in which the adding of the poly A is a step performed in a solution containing Mn²⁺ of 3 mM to 38 mM.
(12) A poly A polymerase reaction solution composition kit including:
   (i) poly A polymerase;
   (ii) a reaction buffer solution that contains Mn²⁺ such that a final concentration of Mn²⁺ is 3 mM to 38 mM in a poly A polymerase reaction solution; and
   (iii) ATP.
(13) A kit of detecting a target oligonucleotide in a sample, in which the target oligonucleotide is an oligonucleotide or a DNA in which a nucleic acid base at a 3'-end is chemically modified, the kit including the followings:
   (i) poly A polymerase
   (ii) a reaction buffer solution that contains Mn²⁺ such that a final concentration of Mn²⁺ is 3 mM to 38 mM in a poly A polymerase reaction solution;
   (iii) a capture probe for capturing the target oligonucleotide; and
   (iv) a pair of self-aggregating probes composed of first and second oligonucleotides.
(14) The kit described in (12), in which the first oligonucleotide contains at least a nucleic acid region X, a nucleic acid region Y, and a nucleic acid region Z or a nucleic acid region Z containing a poly T sequence in order from an 5'-end side, and
   the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.
(15) A pair of self-aggregating probes composed of first and second oligonucleotides, which is used for the detection method of (4) to (7),
   in which the first oligonucleotide contains at least a nucleic acid region X, a nucleic acid region Y, and a nucleic acid region Z or a nucleic acid region Z containing a poly T sequence in order from an 5'-end side, and
   the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.

### ADVANTAGEOUS EFFECTS OF INVENTION

According to the present invention, it is possible to increase the reactivity of a reaction of adding, by poly A polymerase, poly A to an oligonucleotide and a DNA in which the nucleic acid base at the 3'-end is chemically modified. Further, it is possible to increase a detection sensitivity in the case of detection of an oligonucleotide and a DNA in which the nucleic acid base at the 3'-end is chemically modified. Further, it is also possible to detect the nucleic acid from a living body-derived sample such as blood plasma which was originally difficult to detect.

### BRIEF DESCRIPTION OF DRAWINGS

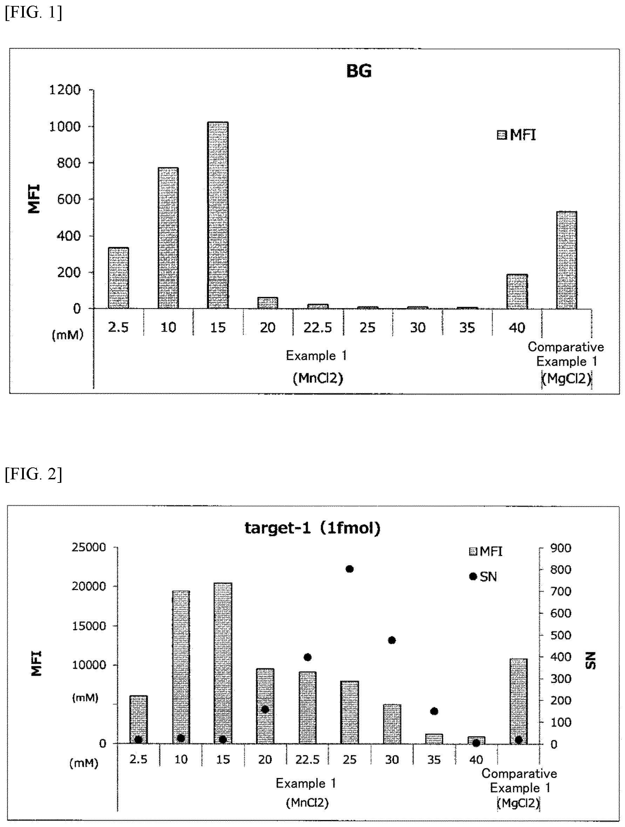

Fig. 1 is a test result obtained by determining the effect of Mn²⁺ when a polyadenylation reaction was performed on a target oligonucleotide Target-1 in a 100% mouse blood plasma matrix, which is a graph illustrating the detection sensitivity (MFI) at each Mn²⁺ concentration for Background (BG, the concentration of the target oligonucleotide is zero) (Example 1). For comparison, the effect of Mg²⁺ was also confirmed (Comparative Example 1).
Fig. 2 is a test result obtained by determining the effect of Mn²⁺ concentration when a polyadenylation reaction was performed on the target oligonucleotide Target-1 in the 100% mouse blood plasma matrix, which is a graph illustrating the detection sensitivity (MFI) at each Mn²⁺ concentration in the case where the concentration of the target oligonucleotide was 1 fmol (Example 1). For comparison, the effect of Mg²⁺ was also confirmed (Comparative Example 1).
Fig. 3 is a test result for determining the effect of Mn²⁺ concentration when a polyadenylation reaction was performed on the target oligonucleotide Target-1 in the 100% mouse blood plasma matrix, which is a graph illustrating the detection sensitivity (MFI) at each Mn²⁺ concentration in the case where the concentration of the target oligonucleotide was 10 fmol (Example 1). For comparison, the effect of Mg²⁺ was also confirmed (Comparative Example 1).
Fig. 4 is a graph illustrating the relationship between the concentration of a target oligonucleotide and the measurement sensitivity when the Mn²⁺ concentration in a polyadenylation reaction solution was 22.5 mM in the case where a polyadenylation reaction was performed on the target oligonucleotide Target-1 in a 100% mouse blood plasma matrix (Example 2).

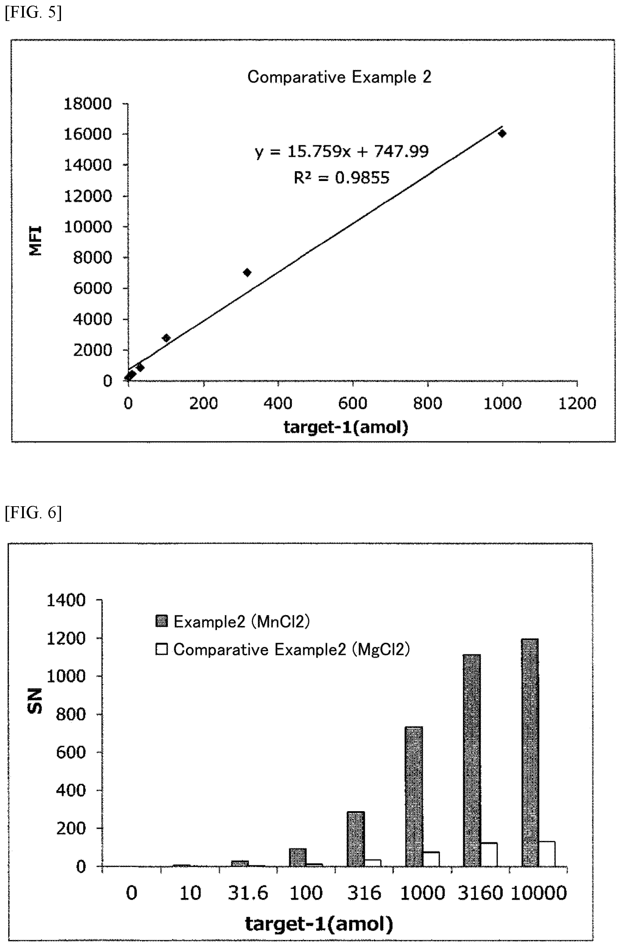

Fig. 5 is a graph illustrating the relationship between the concentration of a target oligonucleotide and the measurement sensitivity when the Mg²⁺ concentration in a polyadenylation reaction solution was 10.0 mM in the case where a polyadenylation reaction was performed on the target oligonucleotide Target-1 in a 100% mouse blood plasma matrix (Comparative Example 2).
Fig. 6 is a graph illustrating the relationship between SN ratios when a polyadenylation reaction was performed on the target oligonucleotide Target-1 in the 100% mouse blood plasma matrix by using a buffer of Example 2 and a buffer of Comparative Example 2 (Example 2).

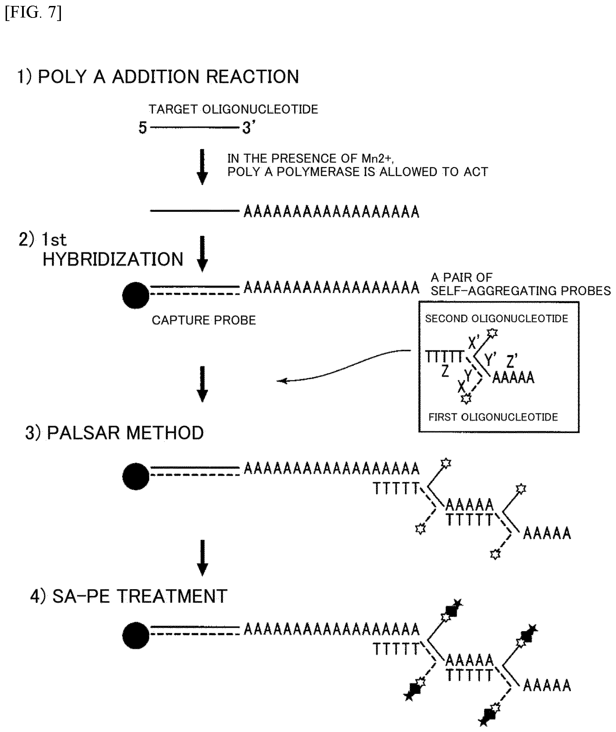

Fig. 7 is a conceptual view illustrating basic steps of polyadenylating a target oligonucleotide, and performing detection by a PALSAR method.

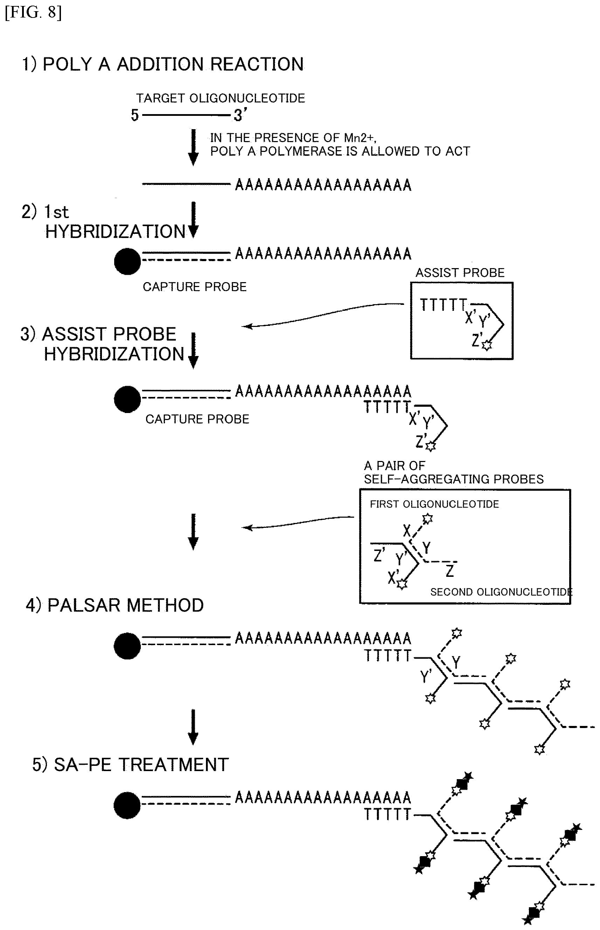

Fig. 8 is a conceptual view illustrating basic steps in the case where an assist probe is used in the steps of performing detection by a PALSAR method after polyadenylating a target oligonucleotide.

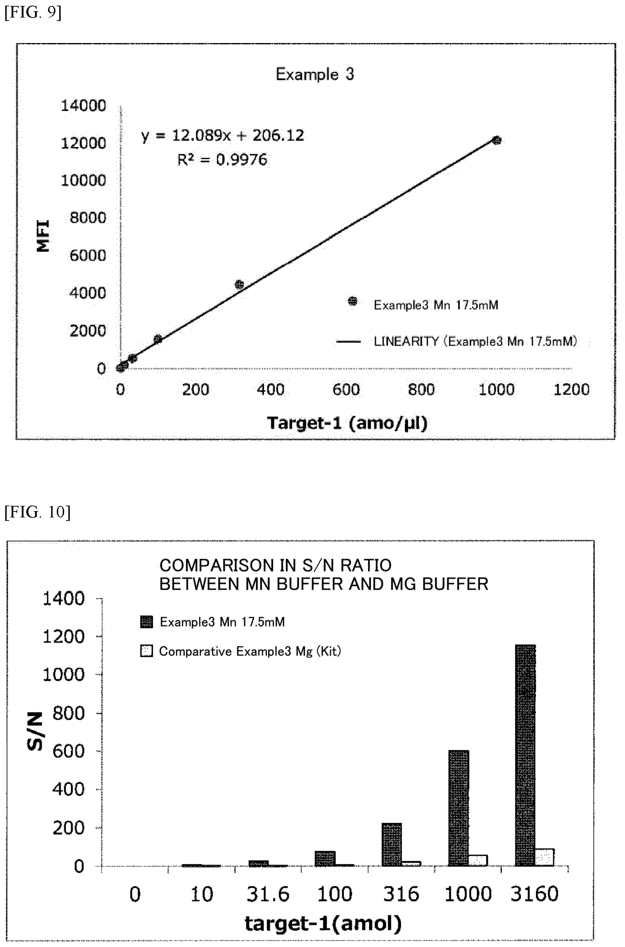

Fig. 9 is a graph illustrating the relationship between the concentration of a target oligonucleotide (Target-1) and the measurement sensitivity when the Mn²⁺ concentration in a polyadenylation reaction solution was 17.5 mM in the case where a polyadenylation reaction was performed on the target oligonucleotide in a 100% mouse blood plasma matrix (Example 3).
Fig. 10 is a graph illustrating the relationship between SN ratios of the target oligonucleotide at each concentration when a polyadenylation reaction was performed on the target oligonucleotide Target-1 in the 100% mouse blood plasma matrix by using a buffer of Example 3 and a buffer of Comparative Example 3.

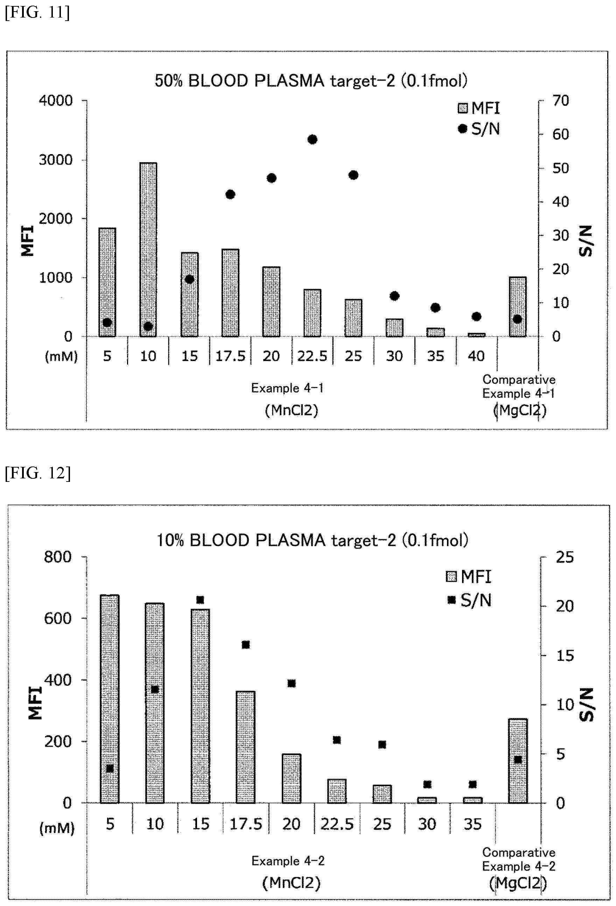

Fig. 11 is a test result obtained by determining the effect of Mn²⁺ concentration when a polyadenylation reaction was performed on a target oligonucleotide Target-2 (3'-end LNA) in a 50% mouse blood plasma matrix, which is a graph illustrating the detection sensitivity (MFI) at each Mn²⁺ concentration in the case where the concentration of the target oligonucleotide was 0.1 fmol (Example 4-1). For comparison, the effect of Mg²⁺ was also confirmed (Comparative Example 4-1).
Fig. 12 is a test result obtained by determining the effect of Mn²⁺ concentration when a polyadenylation reaction was performed on a target oligonucleotide Target-2 (3'-end LNA) in a 10% mouse blood plasma matrix, which is a graph illustrating the detection sensitivity (MFI) at each Mn²⁺ concentration in the case where the concentration of the target oligonucleotide was 0.1 fmol (Example 4-2). For comparison, the effect of Mg²⁺ was also confirmed (Comparative Example 4-2).

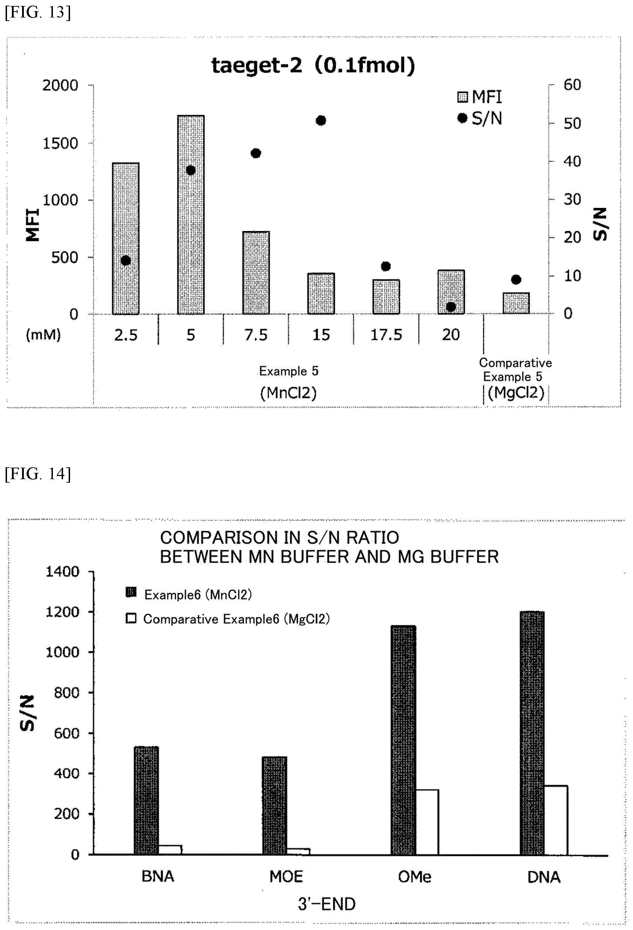

Fig. 13 is a test result obtained by determining the effect of Mn²⁺ concentration when a polyadenylation reaction was performed on a target oligonucleotide Target-2 (3'-end LNA) in water, which is a graph illustrating the MFI at each Mn²⁺ concentration in the case where the concentration of the target oligonucleotide was 0.1 fmol (Example 5). For comparison, the effect of Mg²⁺ was also confirmed (Comparative Example 5).
Fig. 14 is a graph illustrating the comparison between SN ratios in a Mn²⁺ buffer (Example 6) and a Mg²⁺ buffer (Comparative Example 6) when a polyadenylation reaction was performed on target oligonucleotides, Target-3 (3'-end BNA-NC(N-Me)), Target-4 (3'-end MOE), Target-5 (3'-end OMe), and Target-6 (3'-end DNA) in a 100% mouse blood plasma matrix.

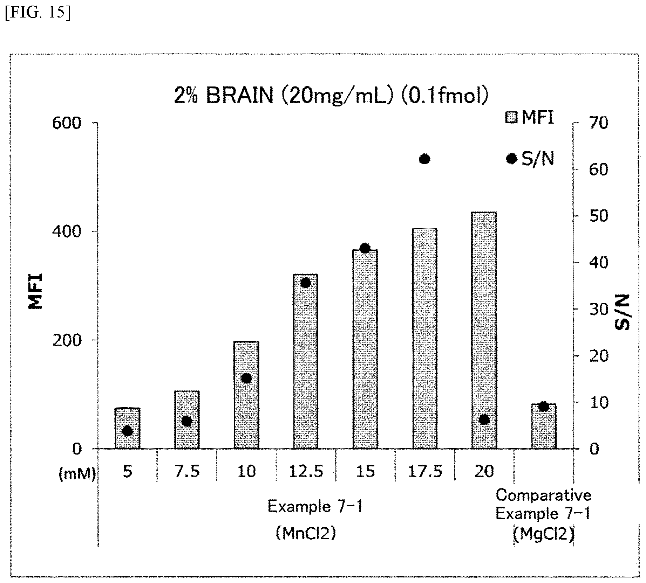

Fig. 15 is a test result obtained by determining the effect of Mn²⁺ when a polyadenylation reaction was performed on a target oligonucleotide Target-2 (3'-end LNA) in a 2% mouse brain matrix (20 mg/mL), which is a graph illustrating the results of the MFI and the SN ratio when the concentration of Target-2 was 0.1 fmol (Example 7-1). For comparison, the effect of Mg²⁺ was also confirmed (Comparative Example 7-1).

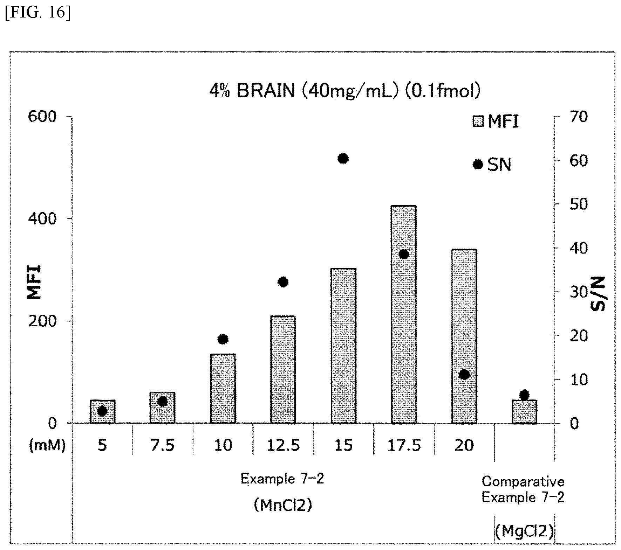

Fig. 16 is a test result obtained by determining the effect of Mn²⁺ when a polyadenylation reaction was performed on a target oligonucleotide Target-2 (3'-end LNA) in a 4% mouse brain matrix (40 mg/mL), which is a graph illustrating the results of the MFI and the SN ratio when the concentration of Target-2 was 0.1 fmol (Example 7-2). For comparison, the effect of Mg²⁺ was also confirmed (Comparative Example 7-2).

### DESCRIPTION OF EMBODIMENTS

### (Poly A addition reaction)

The first aspect of the present invention relates to a reaction of adding poly A to a target oligonucleotide. The poly A addition reaction is a method in which an oligonucleotide or a DNA in which the nucleic acid base at the 3'-end is chemically modified, in a sample, is brought into contact with poly A polymerase (polynucleotide adenyltransferase) so as to add poly A to the 3'-end of the oligonucleotide or the DNA in which the nucleic acid base at the 3'-end is chemically modified. This is a method performed in the presence of Mn²⁺.

The poly A polymerase means a protein having an enzymatic activity that introduces an adenine residue into the 3'-end of single-stranded or double-stranded RNA or DNA. For example, E. coli-derived poly A polymerase is suitably used.

The target oligonucleotide may be polyadenylated by coming into contact with the poly A polymerase and adenosine triphosphate (ATP) under active conditions of the poly A polymerase.

The length of the poly A to be introduced in the present invention is not limited, but, for example, the lower limit is preferably 4 mer or more, more preferably 10 mer or more, further preferably 15 mer or more. The upper limit is preferably 1000 mer or less.

It is desirable that after the addition reaction, the poly A polymerase is inactivated by heat or the like.

In the present specification, unless otherwise specified, the poly A addition, the polyadenylation, and the poly A polymerase reaction are individually shall be used with the same meaning.

### (Collection method)

The second aspect of the present invention relates to a collection method of an oligonucleotide or a DNA in which the nucleic acid base at the 3'-end is chemically modified (hereinafter, also simply referred to as a target oligonucleotide). The present invention includes the following steps (i) to (iii).
(i) is the same as the poly A addition reaction. In this step, in the presence of Mn²⁺, the target oligonucleotide in the sample is brought into contact with the poly A polymerase so as to add poly A to the 3'-end of the target oligonucleotide.
(ii) is a step of bringing the sample into contact with a capture probe for capturing the target oligonucleotide to perform hybridization.

Here, the capture probe includes (A) a nucleic acid probe, and (B) a solid phase, an adapter, or a linker adjacent to a nucleotide at the 3'-end or 5'-end of the nucleic acid probe.

The nucleic acid probe contains an entire sequence or a partial sequence of the target oligonucleotide.
(iii) is a step of collecting a product of hybridization between the target oligonucleotide and the capture probe, which is contained in the sample.

The adjustment of Mn²⁺ concentration in the poly A addition reaction will be described below.

### (Detection method)

The third aspect of the present invention relates to a method of detecting a target oligonucleotide, and includes steps (i) to (iv). (i) to (iii) are the same steps as in the second aspect, and (iv) is a step of detecting the collected hybridization product. The detection method will be described below.

### (Detection by PALSAR method)

The fourth aspect of the present invention relates to a method of detecting a target oligonucleotide through a PALSAR method, and includes steps (i) to (iv). (i) and (ii) are the same steps as in the second aspect.
(iii) is a step of bringing the hybridization product into contact with a pair of self-aggregating probes composed of first and second oligonucleotides so as to form a complex of the hybridization product, and an oligonucleotide polymer obtained through self-aggregation of the first and second oligonucleotides. (iv) is a step of detecting the complex.

The steps (iii) and (iv) are a process that is a so-called PALSAR method (PALSAR method) in which amplification of the self-aggregating probes, and detection of the amplified signal are performed. In some cases, the hybridization product may be directly amplified or in other cases, amplification may be made through an assist probe. When amplification is made without the assist probe, it is desirable that at least one of the self-aggregating probes composed of first and second oligonucleotides of the present invention contains a poly T sequence. Further, when amplification is made through the assist probe, it is desirable that the assist probe contains a poly T sequence, and a sequence complementary to an entire sequence or a partial sequence of at least one of the first and second oligonucleotides. Specific steps of the PALSAR method, the assist probe, and the self-aggregating probes will be further described below.

### (Target oligonucleotide)

The present invention is suitable for detecting nucleic acid therapeutics which have been particularly attracting attention as new molecular targeted drugs in recent years, and are being actively developed. Almost all products of the nucleic acid medical supplies use nucleic acids subjected to some modifications so that degradation in the body may be controlled. Thus, the detection technique of the present invention is suitably applied. For example, a Gapmer structure-type antisense nucleic acid medicine or the like known as an "artificial nucleic acid-DNA-artificial nucleic acid" structure may be exemplified.

Specifically, the target oligonucleotide as a detection target of the present invention is an oligonucleotide or a DNA in which the nucleic acid base at the 3'-end is chemically modified. Examples of the chemical modification include phosphorothioate modification (S-modified), 2'-F modification, 2'-O-Methyl (2'-OMe) modification, 2'-O-Methoxyethyl (2'-MOE) modification, morpholino modification, LNA modification, BNA^{COC} modification, BNA^{NC} modification, ENA modification, and cEt BNA modification.

Among them, as the oligonucleotide in which the nucleic acid base at the 3'-end is chemically modified, LNA, BNA, phosphorothioate, morpholino oligo, boranophosphate, 2'-O-methylated RNA (2'-OMe), 2'-O-methoxyethylated RNA (2'-MOE), or 2'-F is preferred, and LNA, BNA, MOE or OMe is more preferred.

Further, as the DNA, a normal DNA may be exemplified.

The above-mentioned target oligonucleotide and DNA may be either single-stranded or double-stranded. In the case of double strands, these are usually used as a single strand in the present invention, but may also be used as double strands.

### (In the presence of Mn²⁺)

The step of adding the poly A in the present invention needs to be performed in the presence of Mn²⁺. The presence of Mn²⁺ specifically means that the poly A addition reaction is performed in a solution containing Mn²⁺. The concentration of Mn²⁺ in the solution may be appropriately adjusted according to the type of the target oligonucleotide or the type of the sample, the type of a reaction solution, or a desired sensitivity, and is preferably 3 mM to 38 mM, more preferably 5 mM to 35 mM, further preferably 10 mM to 30 mM. When the sample is water or a buffer (a biological sample 0%), the concentration of Mn²⁺ is preferably 3 mM to 17.5 mM, more preferably 5 mM to 15 mM, further preferably 7.5 mM to 15 mM.

When the content of the biological sample (blood plasma, brain, etc.) in the sample is larger than 0% (0 mg/ml) and less than 10% (100 mg/ml), the concentration of Mn²⁺ is preferably 3 mM to 25 mM, more preferably 10 mM to 25 mM, further preferably 10 mM to 20 mM.

When the content of the biological sample (blood plasma, brain, etc.) in the sample is 10% (100 mg/ml) or more and less than 50% (500 mg/ml), the concentration of Mn²⁺ is preferably 7.5 mM to 27.5 mM, more preferably 10 mM to 25 mM, further preferably 12.5 mM to 25 mM.

When the content of the biological sample (blood plasma, brain, etc.) in the sample is 50% (500 mg/ml) or more and less than 100% (1000 mg/ml), the concentration of Mn²⁺ is preferably 12.5 mM to 37.5 mM, more preferably 15 mM to 30 mM, further preferably 15 mM to 25 mM.

When the content of the biological sample (blood plasma, brain, etc.) in the sample is 100% (1000 mg/ml), the concentration of Mn²⁺ is preferably 17 mM to 38 mM, more preferably 17 mM to 35 mM, further preferably 17 mM to 30 mM.

When the biological sample is an object such as a brain, the object is suspended in a suspension, and is stirred with an electrically conductive stirrer or the like to produce a suspension of the object. This is adjusted by being diluted with a diluent as needed. Preferably, adjustment is made so that the object is 0% (0 mg) or more and less than 50% (500 mg/ml).

As the suspension, a general suspension for suspending a biological sample may be exemplified, and examples thereof include a commercially available Direct PCR Lysisi Reagent (VIAGEN biotec) or an RLT buffer (QIAGEN).

As for the diluent, a general diluent may be exemplified, and examples thereof include water, a phosphoric acid buffer, and a tris-hydrochloric buffer.

The solution containing Mn²⁺ is obtained by adding Mn²⁺ to a buffer as described below.

The concentration may be set by performing a reaction between the target oligonucleotide and the poly A polymerase in the presence of Mn²⁺ with different dilution stages, and selecting a concentration at which a desired activity is obtained. The desired activity may be evaluated by measuring the uptake amount of AMP by using ATP in the reaction step as a substrate, or by performing a PALSAR reaction after the step and detecting an amplification signal.

In the present invention, the poly A addition reaction is a reaction in which poly A is added to the target oligonucleotide to form an adduct thereof (hereinafter, also simply referred to as "target oligonucleotide-poly A"). Then, through hybridization between the adduct and the capture probe, double strands of the "target oligonucleotide-poly A" and the capture probe are formed.

### (Capture probe)

The "capture probe" in the present invention is a probe for capturing the target oligonucleotide, and includes (A) a nucleic acid probe, and (B) a solid phase, an adapter, or a linker adjacent to a nucleotide at the 3'-end or 5'-end of the nucleic acid probe.

### (Nucleic acid probe)

The nucleic acid probe (A) used in the present invention may be a substance that may be specifically bound to the target oligonucleotide, and is not particularly limited, but contains a base sequence complementary to the target oligonucleotide.

As the solid phase (B), for example, it is desirable to use a substrate such as insoluble fine particles, microbeads, fluorescent fine particles, magnetic particles, microplates, microarrays, a slide glass, or an electrically conductive substrate.

The "nucleic acid probe" used in the present invention contains a sequence complementary to an entire sequence or a partial sequence of the target oligonucleotide. It is desirable to contain a sequence complementary to a portion including the 3'-end or the 5'-end of the target oligonucleotide with respect to the total length of the target oligonucleotide. Following cases (a-1) and (a-2) may be exemplified.
(a-1) when the solid phase, or the adapter or the linker is adjacent to a nucleotide at the 3'-end, the nucleic acid probe contains a sequence complementary to the target oligonucleotide in a portion including the 3'-end nucleotide.
(a-2) when the solid phase, or the adapter or the linker is adjacent to a nucleotide at the 5'-end, the nucleic acid probe contains a sequence complementary to the target oligonucleotide in a portion including the 5'-end nucleotide.

In the nucleic acid probe, the sequence complementary to a portion including the 3'-end or the 5'-end of the target oligonucleotide preferably contains a sequence complementary to a portion of the target oligonucleotide.

Further, the sequence complementary to the total length of the target oligonucleotide may have sameness to the extent that hybridization may be made for the total length, and is more preferably a sequence completely complementary to the same total length.

Further, since poly A is added to the 3'-end of the target oligonucleotide, the nucleic acid probe may contain polybase T (or U) adjacent to the 5'-end nucleotide of the sequence complementary to the target oligonucleotide.

The length (the number of bases) of the sequence of the nucleic acid probe only has to be a length by which binding to the target oligonucleotide may be made. For example, the lower limit of the bases is preferably 4 mer or more, more preferably 10 mer or more, further preferably 15 mer or more. The upper limit of the bases is preferably 1000 mer or less.

### (Solid phase)

As the solid phase in the present invention, a substrate such as insoluble fine particles, microbeads, fluorescent fine particles, magnetic particles, microplates, microarrays, a slide glass, or an electrically conductive substrate may be exemplified. Fluorescent fine particles are preferred, fluorescent beads are more preferred, and beads having a fluorescent substance on the surfaces thereof are particularly preferred. The "beads having a fluorescent substance on the surfaces thereof" used in the present invention are not particularly limited as long as they are beads having a fluorescent substance. For example, MicroPlex^{™} Microspheres of Luminex may be appropriately used. One type of beads may be used, or many types of beads may be used. When a plurality of types of color-coded beads is used, the oligonucleotide quantification method of the present invention may also be easily multiplexed.

The solid phase and the nucleic acid probe may be directly bound, or may be bound via an adapter or a linker. Preferably, it is desirable that binding is made via the adapter or the linker.

In the present invention, "adjacent" in "the solid phase adjacent to a nucleotide at the 3'-end or 5'-end of the nucleic acid probe" means direct binding to the nucleotide, or binding via an adapter, a linker or the like, and the meaning of binding will be described below.

### (Adapter or linker)

Examples of the "adapter or linker" used in the present invention include compounds having an amino group or a carboxyl group, such as biotin or spacers such as Spacer 9, Spacer 12, Spacer 18, and Spacer C3. 5'-Amino-Modifier C12(12-(4-Monomethoxytritylamino)dodecyl-1-[(2-cyanoethyl)-(N,N-diisopropyl)]-p hosphoramidite), and biotin are preferred.

For example, in a mode in which a carboxyl group is contained in the bead surface, and a nucleic acid probe to which a compound of an amino group is added is bound to the carboxyl group on the bead surface via the amino group, the compound having the amino group becomes an example of a linker.

### (Label substance)

As for the label substance in the present invention, suitable examples include a radioisotope, biotin, digoxigenin, a fluorescent substance, a luminescent substance and a dye.

Specifically, it is also possible to detect the target oligonucleotide by adding a donor fluorescent dye and an acceptor fluorescent dye for using fluorescent resonance energy transfer (FRET), such as a radioisotope such as ¹²⁵I or ³²P, a luminescent or coloring substance such as digoxigenin or acridinium ester, alkaline phosphatase for using a luminescent substance such as dioxetane or a fluorescent substance such as 4-methylumbelliferylphosphate, and biotin for using a fluorescent, luminescent, or coloring substance, etc. bound to avidin.

### (Binding of nucleic acid probe to solid phase)

For the binding of the nucleic acid probe to the solid phase, a method by chemical bonding, a method by biological interaction, a method by physical adsorption, etc. may be exemplified. In the method by chemical bonding, for example, when a carrier coated with a carboxyl group is used, a coupling reaction may be carried out with an amino group labeled on an oligonucleotide. In the method by biological interaction, for example, a binding force between streptavidin coated on a carrier and biotin for modification of the nucleic acid probe may be used. Further, in the method by physical adsorption, for example, when the solid phase having a negative charge is used, the oligonucleotide may be labeled with a substance having a positive charge such as an amino group and then may be electrostatically adsorbed on the carrier.

### (Contact)

In the present specification, the term "brining in contact with," or "contact step" means that one substance and another substance are placed close to each other such that a chemical bond such as a covalent bond, an ionic bond, a metal bond, or a non-covalent bond may be formed between these substances. In one aspect of the present invention, "bringing one substance into contact with another substance" means that a solution containing one substance is mixed with a solution containing another substance. The step of bringing the sample in contact with the poly A polymerase and ATP is performed by keeping the temperature warm at 30 to 42°C for 5 to 120 min, preferably keeping the temperature warm at 37°C for 60 min. The step of bringing the sample in contact with the capture probe is performed by keeping the temperature warm at 30 to 70°C for 0.2 to 30h, preferably keeping the temperature warm at 35 to 65°C for 8 to 24h. The step of bringing the sample in contact with first and second oligonucleotides is performed by keeping the temperature warm at 28 to 70°C for 0.2 to 3h, preferably keeping the temperature warm at 30 to 54°C for 0.5 to 2h.

Further, the contact between the target oligonucleotide and the capture probe means that the contact is performed under a condition in which corresponding bases of complementary sequences of the target oligonucleotide and the nucleic acid probe may be hybridized. Similarly, the contact between the pair of self-aggregating probes, the capture probe, and the assist probe, or the contact between paired self-aggregating probes also means that the contact is performed under a condition in which corresponding bases may be hybridized.

### (Self-aggregation)

In the present specification, the term "self-aggregation" means a state where a plurality of first oligonucleotides forms a complex through hybridization with a second oligonucleotide, and a state where a plurality of second oligonucleotides forms a complex through hybridization with a first oligonucleotide.

### (Pair of self-aggregating probes)

The pair of "self-aggregating" probes used in the method of the present invention refers to oligonucleotides which have complementary base sequence regions where the first oligonucleotide and the second oligonucleotide may hybridize with each other and are capable of forming a probe polymer through a self-aggregating reaction. Preferably, at least one of the first and second oligonucleotides is labeled with a label substance. Here, "hybridizable" means, in one aspect, complete complementariness in the complementary base sequence regions.

The pair of self-aggregating probes may also be labeled with a label substance for detection in advance. As for such a label substance, suitable examples include a radioisotope, biotin, digoxigenin, a fluorescent substance, a luminescent substance, and a dye. Specifically, it is also possible to detect the target oligonucleotide by adding a donor fluorescent dye and an acceptor fluorescent dye for using fluorescent resonance energy transfer (FRET), such as a radioisotope such as ¹²⁵I or ³²P, a luminescent or coloring substance such as digoxigenin or acridinium ester, alkaline phosphatase for using a luminescent substance such as dioxetane or a fluorescent substance such as 4-methylumbelliferylphosphate, and biotin for using a fluorescent, luminescent, or coloring substance, etc. bound to avidin.

Preferably, the label substance is biotin, and labelling of the oligonucleotide is preferably performed by biotinylating the 5'-end or the 3'-end. When the label substance is biotin, a substance specifically bound to the label substance is streptavidin or avidin.

In the more specific description on the pair of "self-aggregating" probes, the first oligonucleotide is an oligonucleotide containing at least a nucleic acid region X, a nucleic acid region Y and a nucleic acid region Z in order from the 5'-end side, and the second oligonucleotide is an oligonucleotide containing at least a nucleic acid region X' complementary to the nucleic acid region X, a nucleic acid region Y' complementary to the nucleic acid region Y, and a nucleic acid region Z' complementary to the nucleic acid region Z in order from the 5'-end side.

When the PALSAR method is performed without the assist probe to be described below, a mode in which the nucleic acid region Z contains a poly T sequence and the nucleic acid region Z' contains a poly A sequence is exemplified.

In the present invention, in the step of bringing the sample containing the hybridization product of the "target oligonucleotide-poly A" and the capture probe, in contact with the pair of self-aggregating probes composed of the first and second oligonucleotides so as to form a complex with the oligonucleotide polymer composed of the first and second oligonucleotides, there are cases where the complex is formed without the assist probe, and the complex is formed via the assist probe.

The assist probe is a probe having a role of assisting in forming the complex of the target oligonucleotide-poly A and the oligonucleotide polymer. In the first aspect, the assist probe is a probe that contains a sequence complementary to an entire sequence or a partial sequence of at least one of the first and second oligonucleotides, and an entire sequence or a partial sequence of the target oligonucleotide.

The assist probe may be labeled with a label substance, or may not be labeled.

Further, the assist probe of the present invention is a probe that contains an entire sequence or a partial sequence of at least one of the first and second oligonucleotides, and a sequence complementary to an entire sequence or a partial sequence of poly A added to the target oligonucleotide (that is, poly T).

For example, when the sequence of one of the paired self-aggregating probes is XYZ, (poly T)-X'Y'X' and (poly T)-Z'Y'Z' may be exemplified.

Hereinafter, the cases where the assist probe is present and is not present will be separately described.

### (Case where PALSAR method is performed without assist probe)

When the complex with the pair of self-aggregating probes is formed without the assist probe, at least one of the first and second oligonucleotides may contain a complementary strand (poly T) of an entire sequence or a partial sequence of poly A added to the target oligonucleotide. It is desirable that at least one of the first and second oligonucleotides is labeled with a label substance.

In the first aspect of the detection method of the target oligonucleotide of the present invention, a detection method performed by a PALSAR method after poly A is added to the target oligonucleotide will be specifically described below with reference to Fig. 7.

First, a sample that may contain a target oligonucleotide and a capture probe for capturing the target oligonucleotide are prepared.

The capture probe of the present invention includes
(A) a nucleic acid probe, and
(B) beads immobilized on a nucleotide portion at the 3'-end of the nucleic acid probe.

The nucleic acid probe contains the entire sequence of the target oligonucleotide.

Next, poly A polymerase is allowed to act on the 3'-end of the target oligonucleotide in the sample so as to add poly A (1) in Fig. 7). The present invention is characterized in that in this step, the Mn²⁺ concentration in the reaction solution is adjusted to a specific concentration so as to increase the addition reactivity of poly A, and then to increase the detection sensitivity by the subsequent PALSAR reaction.

Subsequently, the capture probe is brought into contact with the target oligonucleotide to which poly A is added (1^{st} hybridization). Accordingly, the target oligonucleotide to which poly A is added is hybridized with the nucleic acid probe in the capture probe to form double strands (2) in Fig. 7). The target oligonucleotide included in the hybridization product may be quantified by the following PALSAR method.

The sample including the hybridization product is brought into contact with a pair of self-aggregating probes composed of first and second oligonucleotides to form a complex of the hybridization product, and an oligonucleotide polymer obtained through self-aggregation of the first and second oligonucleotides (a so-called PALSAR reaction).

Here, in the pair of self-aggregating probes composed of the first and second oligonucleotides, the first oligonucleotide is an oligonucleotide that contains a nucleic acid region X, a nucleic acid region Y, and a nucleic acid region Z in order from the 5'-end side, and the second oligonucleotide contains a nucleic acid region X' complementary to the nucleic acid region X, a nucleic acid region Y' complementary to the nucleic acid region Y, and a nucleic acid region Z' complementary to the nucleic acid region Z in order from the 5'-end side (hereinafter, the nucleic acid regions will be also simply referred to as X, Y, Z, X', Y', and Z').

Further, Z contains a poly T sequence, and Z' contains a poly A sequence. Further, a label substance is attached to the 5'-end of the first and second oligonucleotides.

Z (poly T) of the first oligonucleotide is hybridized with poly A of the hybridization product, and X' and Y' of the second oligonucleotide are hybridized with X and Y of the first oligonucleotide, respectively. Then, X and X', Y and Y', and Z (poly T) and Z' (poly A) are repeatedly hybridized one after another so as to form the oligonucleotide polymer of the self-aggregating probes (3) in Fig. 7).

The complex of the hybridization product of the capture probe and the target oligonucleotide and the first and second self-aggregated oligonucleotides may be precipitated or separated by centrifugation so that the label substance or the like of the first and second oligonucleotides may be detected. Then, the concentration or the like of the target oligonucleotide may be measured (4) in Fig. 7).

It is also possible to detect the target oligonucleotide by detecting a double-stranded hybridization product formed by the capture probe and the poly A-added target oligonucleotide before the PALSAR reaction is performed. For example, a poly T probe whose 5'-end is labeled with a label substance in advance may be hybridized with the poly A region of the double-stranded hybridization product so that the label substance of the poly T probe may be detected. Then, the target oligonucleotide may be detected.

### (Case where PALSAR method is performed via assist probe)

When the complex is formed via the assist probe, the first or second oligonucleotide may be an oligonucleotide capable of forming a probe polymer through a self-aggregating reaction. In this case, the assist probe may contain poly T, and an entire sequence or a partial sequence of at least one of the first and second oligonucleotides. At least one of the first oligonucleotide, the second oligonucleotide, and the assist probe is preferably labeled with a label substance.

In the second aspect of the detection method of the target oligonucleotide of the present invention, a detection method performed through an assist probe after poly A is added to the target oligonucleotide will be specifically described below with reference to Fig. 8.

The step of adding poly A and the step of hybridizing "poly A-target oligonucleotide" with a capture probe are the same as in the case where the PALSAR method is performed without the assist probe.

The sample including the hybridization product is brought into contact with the assist probe (3) assist probe hybridization in Fig. 8). Here, the assist probe contains a poly T sequence and a sequence of a second oligonucleotide (X', Y', and Z' or X', Y', and X'). The poly T sequence of the assist probe is hybridized with poly A of the "target oligonucleotide-poly A" to form a complex of the "target oligonucleotide-poly A" and the assist probe.

This is brought into contact with a pair of self-aggregating probes composed of first and second oligonucleotides to form a complex of the complex and an oligonucleotide polymer obtained through self-aggregation of the first and second oligonucleotides (a so-called PALSAR reaction).

In the pair of self-aggregating probes composed of the first and second oligonucleotides, the first oligonucleotide is an oligonucleotide that contains X, Y and Z in order from the 5'-end side, and the second oligonucleotide contains X', Y' and Z' in order from the 5'-end side. A label substance is attached to the 5'-end of the first and second oligonucleotides. X', Y', and Z' of the assist probe are hybridized with X, Y, and Z of the first oligonucleotide, and the first oligonucleotide is hybridized with the second oligonucleotide. Then, X and X', Y and Y', and Z and Z' are repeatedly hybridized one after another so as to form the oligonucleotide polymer of the self-aggregating probes (4) in Fig. 8). The detection method of such a complex is the same as in the case where the assist probe is not present.

The specific example of the PALSAR method is illustrated in Fig. 4 to Fig. 14 of International Publication No. 2013/172305, which may be appropriately modified on the basis of the common sense of those skilled in the art by using a dimer forming probe or the like and then may be applied to the self-aggregating reaction of the present invention.

The capture probe of the present invention and the polymer of the first and second self-aggregated oligonucleotides (signal probe polymer) may be preferably separated. The separation method of the complex is not particularly limited, and preferred examples include a centrifugal method, and a suction filtration method.

The step of precipitating the capture probe and its complex by the "centrifugal method" is usually performed at 20 to 30°C for 0.2 to 5 min through centrifugation of 500 to 3000xg, at 23 to 28°C for 0.5 to 2 min through centrifugation of 800 to 1500xg, preferably at 25°C for 1 min through centrifugation of 1000×g. More specifically, this may follow the instruction manual of the bead manufacturer.

The step of separating the capture probe and its complex by the "suction filtration method," may be carried out by the method described in Biochem Biophys Res Commun. 2015 Nov 27; 467(4): 1012-8. Specifically, a solution containing the capture probe and its complex is transferred to a filter plate, and is usually aspirated at 20 to 30°C in a negative pressure range of 1 to 10 in.Hg, aspirated at 23 to 28°C at 1 to 10 in.Hg, and preferably aspirated at 25°C at 1 to 5 in.Hg. The suction time may be such that the liquid is visually removed from the filter, and examples thereof include 1 sec to 5 min, preferably 5 sec to 1 min. The filter plate having a pore size smaller than the diameter of the capture probe is appropriately used, and for example, 1.2 µm or 1.0 µm is preferred. Specific examples of components include: a filter plate: MultiScreen^{(registered trademark)} HTS-BV plate (Merck Millipore, product number: MSBVN1250), a manifold: MultiScreen^{(registered trademark)} HTS Vacuum Manifold (Merck Millipore), and a suction pump: Chemical duty pump (Merck Millipore, catalog number: WP6110060).

### (Sample)

Examples of the "sample" used in the method of the present invention include biological samples such as body fluids of humans, monkeys, dogs, pigs, rats, guinea pigs or mice, e.g., whole blood, blood serum, blood plasma, lymph, urine, saliva, tears, sweat, gastric juice, pancreatic juice, bile, pleural effusion, articular cavity fluid, cerebrospinal fluid, spinal fluid, and bone marrow fluid, or tissues, e.g., brain, liver, kidney, lung, and heart. Preferably, the sample is whole blood, blood serum, blood plasma, brain, or urine of a human. More preferably, the sample is whole blood, blood serum, blood plasma, brain or urine of a human who has been administered a drug containing the target oligonucleotide. These may be used by being diluted with water or a buffer.

Further, the sample of the present invention also contains the target oligonucleotide diluted with water or a buffer.

The buffer may be any commonly used buffer, and examples thereof include tris hydrochloric acid, boric acid, phosphoric acid, acetic acid, citric acid, succinic acid, phthalic acid, glutaric acid, maleic acid, glycine, and salts thereof, and Good's buffers such as MES, Bis-Tris, ADA, PIPES, ACES, MOPSO, BES, MOPS, TES, and HEPES. Examples of the water include RNase, and DNase free water.

Examples of the detection method of the polymer of the first and second oligonucleotide of the present invention include a turbidity method, an agarose electrophoresis method, an absorbance method, a fluorescence measurement method, an electrochemical luminescence method, and a flow cytometry method. Preferably, a flow cytometry method may be exemplified.

The flow cytometry method is performed, for example, in the "step of detecting the first fluorescence emitted by a first fluorescent substance and the second fluorescence emitted by a second fluorescent substance by using the flow cytometry method." In the "step of detecting the first fluorescence emitted by the first fluorescent substance and the second fluorescence emitted by the second fluorescent substance by using the flow cytometry method," it is desirable to detect the first fluorescence emitted by the first fluorescent substance and the second fluorescence emitted by the second fluorescent substance at the same time or at the same opportunity. Here, "to detect at the same time or at the same opportunity" means that the first fluorescence and the second fluorescence, which are emitted by simultaneous or sequential excitation of the first fluorescent substance and the second fluorescent substance included in one complex (a three-component complex or a four-component complex) within a flow path of a flow cytometer, are detected as fluorescence emitted from the one complex.

More specifically, examples of the first fluorescent substance include a fluorescent substance contained in the solid phase of the capture probe, and examples of the second fluorescent substance include a fluorescent substance previously bound to a substance that is specifically bound to the label substance attached to the first and second oligonucleotides. For a reaction solution containing these first and second fluorescent substances, it is possible to detect the first fluorescence emitted by the first fluorescent substance and the second fluorescence emitted by the second fluorescent substance by using the flow cytometry method.

In the present invention, a poly A polymerase buffer kit includes at least following components.
(1) poly A polymerase
(2) A reaction buffer solution that contains Mn²⁺ such that Mn²⁺ is 3 mM to 38 mM in a final reaction solution
(3) ATP

The (2) reaction buffer solution may have a concentration such that the concentration of Mn²⁺ is 3 mM to 38 mM in the final reaction solution in which a sample, each reagent of the kit, etc. are added, preferably 5 mM to 35 mM, more preferably 10 mM to 30 mM. For example, in the case of a two-fold solution (x2), the concentration of Mn²⁺ may be 6 mM to 76 mM, and is preferably 10 mM to 70 mM, more preferably 20 mM to 60 mM. In the case of a three-fold solution (x3), the concentration of Mn²⁺ may be 9 mM to 114 mM, and is preferably 15 mM to 105 mM, more preferably 30 mM to 90 mM. In the case of a four-fold solution (x4), the concentration of Mn²⁺ may be 12 mM to 152 mM, and is preferably 20 mM to 140 mM, more preferably 40 mM to 120 mM. In the case of a five-fold solution (x5), the concentration of Mn²⁺ may be 15 mM to 190 mM, and is preferably 25 mM to 175 mM, more preferably 50 mM to 150 mM. In the case of a six-fold solution (x6), the concentration of Mn²⁺ may be 18 mM to 228 mM, and is preferably 30 mM to 210 mM, more preferably 60 mM to 180 mM. In the case of a seven-fold solution (x7), the concentration of Mn²⁺ may be 21 mM to 266 mM, and is preferably 35 mM to 245 mM, more preferably 70 mM to 210 mM. In the case of an eight-fold solution (x8), the concentration of Mn²⁺ may be 24 mM to 304 mM, and is preferably 40 mM to 280 mM, more preferably 80 mM to 240 mM. In the case of a nine-fold solution (x9), the concentration of Mn²⁺ is 27 mM to 342 mM, preferably 45 mM to 315 mM, more preferably 90 mM to 270 mM. In the case of a ten-fold solution (×10), the concentration of Mn²⁺ may be 30 mM to 380 mM, and is preferably 50 mM to 350 mM, more preferably 100 mM to 300 mM.

The buffer may be any buffer as long as the activity of the poly A polymerase is kept, and in general, tris hydrochloric acid, phosphoric acid, etc. are used.

### (Description on kit in the case of component measurement in water or buffer)

When the poly A polymerase buffer kit measures a component in water or a buffer, the (2) reaction buffer contains Mn²⁺ such that Mn²⁺ is 3 mM to 17.5 mM in a final reaction solution.

When a component in water or the buffer is measured, the (2) reaction buffer solution may have a concentration such that the concentration of Mn²⁺ is 3 mM to 17.5 mM in the final reaction solution in which a sample, each reagent of the kit, etc., are added. The concentration is preferably 5 mM to 15 mM, more preferably 7.5 mM to 15 mM. For example, in the case of a two-fold solution (x2), the concentration of Mn²⁺ is 6 mM to 35 mM, preferably 10 mM to 30 mM, more preferably 15 mM to 30 mM. In the case of a three-fold solution (x3), the concentration of Mn²⁺ may be 9 mM to 52.5 mM, and is preferably 15 mM to 45 mM, more preferably 22.5 mM to 45 mM. In the case of a four-fold solution (x4), the concentration of Mn²⁺ may be 12 mM to 70 mM, and is preferably 20 mM to 60 mM, more preferably 30 mM to 60 mM. In the case of a five-fold solution (x5), the concentration of Mn²⁺ may be 15 mM to 87.5 mM, and is preferably 25 mM to 75 mM, more preferably 37.5 mM to 75 mM.

In the case of a six-fold solution (x6), the concentration of Mn²⁺ may be 18 mM to 105 mM, and is preferably 30 mM to 90 mM, more preferably 45 mM to 90 mM. In the case of a seven-fold solution (x7), the concentration of Mn²⁺ may be 21 mM to 122.5 mM, and is preferably 35 mM to 105 mM, more preferably 52.5 mM to 105 mM. In the case of an eight-fold solution (x8), the concentration of Mn²⁺ may be 24 mM to 140 mM, and is preferably 40 mM to 120 mM, more preferably 60 mM to 120 mM.

In the case of a nine-fold solution (x9), the concentration of Mn²⁺ may be 27 mM to 157.5 mM, and is preferably 45 mM to 135 mM, more preferably 67.5 mM to 135 mM. In the case of a ten-fold solution (x10), the concentration of Mn²⁺ may be 30 mM to 175 mM, and is preferably 50 mM to 150 mM, more preferably 75 mM to 150 mM.

### (Description on kit in the case of component measurement of biological sample 100% (a blood sample 100% or the like))

When the poly A polymerase buffer kit measures a biological sample 100% (a blood sample 100% or the like), the (2) reaction buffer contains Mn²⁺ such that Mn²⁺ is 17 mM to 38 mM in a final reaction solution.

When a component of the biological sample 100% (the blood sample 100% or the like) is measured, the (2) reaction buffer solution may have a concentration such that the concentration of Mn²⁺ is 17 mM to 38 mM in the final reaction solution in which a sample, each reagent of the kit, etc. are added. The concentration is preferably 17 mM to 35 mM, more preferably 17 to 30 mM. For example, in the case of a two-fold solution (x2), the concentration of Mn²⁺ may be 34 mM to 76 mM, and is preferably 34 mM to 70 mM, more preferably 34 mM to 60 mM. In the case of a three-fold solution (x3), the concentration of Mn²⁺ may be 51 mM to 114 mM, and is preferably 51 mM to 105 mM, more preferably 51 mM to 90 mM. In the case of a four-fold solution (x4), the concentration of Mn²⁺ may be 68 mM to 152 mM, and is preferably 68 mM to 140 mM, more preferably 68 mM to 120 mM. In the case of a five-fold solution (x5), the concentration of Mn²⁺ may be 85 mM to 190 mM, and is preferably 85 mM to 175 mM, more preferably 85 mM to 150 mM. In the case of a six-fold solution (x6), the concentration of Mn²⁺ may be 102 mM to 228 mM, and is preferably 102 mM to 210 mM, more preferably 102 mM to 180 mM. In the case of a seven-fold solution (x7), the concentration of Mn²⁺ may be 119 mM to 266 mM, and is preferably 119 mM to 245 mM, more preferably 119 mM to 210 mM. In the case of an eight-fold solution (x8), the concentration of Mn²⁺ may be 136 mM to 304 mM, and is preferably 136 mM to 280 mM, more preferably 136 mM to 240 mM. In the case of a nine-fold solution (x9), the concentration of Mn²⁺ may be 153 mM to 342 mM, and is preferably 153 mM to 315 mM, more preferably 153 mM to 270 mM. In the case of a ten-fold solution (x10), the concentration of Mn²⁺ may be 170 mM to 380 mM, and is preferably 170 mM to 350 mM, more preferably 170 mM to 300 mM.

Further, the kit for detecting the target oligonucleotide of the present invention contains the following (3) and (4), as well as the above (1) and (2).
(3) A capture probe for capturing the target oligonucleotide
(4) A pair of self-aggregating probes composed of first and second oligonucleotides

Further, as necessary, an assist probe or the like is contained. The description on each component is the same as above.

The reaction buffer may be precipitated in some cases when stored for a long period of time, and thus preparation at the time of use is preferred, and an oxidation-reducing agent such as DTT, or a chelating agent such as EDTA may be added.

The concentration of the oxidation-reducing agent or the chelating agent is preferably 0.01 mM to 5 mM, more preferably 0.5 mM to 2 mM.

Further, the oxidation-reducing agent, and the reducing agent may be added in the step of the poly A addition reaction.

### EXAMPLES

### [Example 1] About MnCl₂ concentration during poly A addition reaction by poly A polymerase (100% mouse blood plasma matrix)

### (1) Poly A addition reaction

### (1-1) Preparation of sample

Target-1 (target oligonucleotide) having a sequence illustrated below was adjusted with mouse blood plasma (Charles River, Japan) so that a final concentration became a concentration of 0, 1, and 10 fmol.
<Sequence of target-1>
   T(L)^G(L)^A(L)^g^c^t^g^a^c^t^t^g^a^T(L)^G(L)^5(L) (SEQ ID No. 1 regarding a base sequence part)
"(L)" indicates "LNA."
"5" indicates "5-methyl cytosine."
"^" indicates "S(phosphorothioate)-modified ."

### (1-2) Preparation of poly A polymerase reaction solution

(i) Preparation of PAP Mix by commercially available kit (Comparative Example 1)

A reagent attached with an A-Plus^{™} Poly (A) Polymerase Tailing Kit (CELLSCRIPT, product number: C-PAP5104H) was used. Then, a total of 35 µL of PAP Mix was prepared by mixing 4 µL of 10× reaction buffer (attached to the kit; 0.5 M Tris-HCl (pH 8.0), 2.5 M NaCl, and 100 mM MgCl₂), 4 µL of 10 mM ATP (attached to the kit), 1 µL of poly(A) polymerase (attached to the kit), and 26 µL of Nuclease-Free Water. When the PAP Mix attached to the kit was used, the final concentration of MgCh in the poly A polymerase anti-solution was 10 mM.

### (ii) Preparation of PAP Mix of the present invention (Example 1)

In order to add poly A to the 3'-end of Target-1, a PAP reaction was carried out by using a self-prepared 5x PAP Mn buffer (0.25 M Tris-HCl (pH 8.0), 1.25 M NaCl, and MnCl₂). The reaction solution was adjusted by using a PCR plate so as to obtain a final concentration of 1× PAP Mn buffer, and 1 mM ATP in a total volume of 40 µL. Further, 4 U of poly A polymerase was added. Regarding MnCl₂ concentration, a 5x PAP Mn buffer was prepared such that the concentration in a 1× PAP Mn buffer became 2.5, 10, 15, 20, 22.5, 25, 30, 35, and 40 mM, and then was used for the experiment.

### (1-3) Poly A addition reaction

35 µL of the PAP Mix and 5 µL of the target-1 solution were added to a PCR plate and were mixed (40 µL in total). By using a thermal cycler (Mastercycler nexus GSX1 manufactured by Eppendorf), the reaction solution was kept warm at 37°C for 60 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) having the following sequence complementary to target-1 was bound to MicroPlex^{™} Microspheres of Luminex (Region No.: 43, product number: LC10043-01), through NH₂ modification at the 3'-end (referred to as measurement target substance capturing beads).

### <Sequence of nucleic acid probe CP-1>

### gcatcaagtcagctca (SEQ ID No. 2)

### (2-2) Composition of 1^{st} hybridization reaction solution

Preparation was carried out to obtain a final concentration of 1.6x supplement (10× supplement; [500 mM Tris-HCl (Ph 8.0), 40 mM EDTA (pH 8.0), 8% N-sodium lauroyl sarcosinate]), 1.1 M tetramethylammonium chloride (hereinafter, referred to as TMAC) (SIGMA, T3411), and 500 measurement target substance capturing beads prepared in the (2-1), in 65 µL obtained through mixing with the PAP reaction solution.

### (2-3) 1st hybridization reaction

25 µL of the 1st hybridization reaction solution was added to the oligonucleotide solution obtained after the poly (A) addition reaction, and mixing was performed (65 µL in total). The mixed solution was kept warm at 42°C for 1 h, and then was kept at 4°C.

### (3) Formation reaction of a complex for detection (self-aggregating reaction/PALSAR reaction)

### (3-1) Preparation of PALSAR reaction solution

Addition was carried out to obtain a final concentration of 1.6x supplement and 2.1 M TMAC, in 100 µL obtained through mixing with the 1st hybridization reaction solution. As a pair of self-aggregating probes, HCP-1 (SEQ ID No. 3), and HCP-2 (SEQ ID No. 4) having sequences illustrated below, in which the 5'-end was labeled with biotin, were used. HCP-1 and HCP-2 were added so that their respective final concentrations became 0.7 pmol/µL, and 0.56 pmol/µL, respectively.
<Sequence of HCP-1>
   5'-(biotin)CAACAATCAGGAC GATACCGATGAAG TTTTTTTTTTTTTTTTTTTT-3' (SEQ ID No. 3 regarding a base sequence part)
<Sequence of HCP-2>
   5'-(biotin) GTCCTGATTGTTG CTTCATCGGTATC AAAAAAAAAAAAAAAAAAAA-3' (SEQ ID No. 4 regarding a base sequence part)

### (3-2) Formation reaction of complex for detection (self-aggregating reaction/PALSAR reaction)

35 µL of the PALSAR reaction solution was added to 65 µL of the reaction solution obtained after the 1st hybridization step to make a total of 100 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× pbs (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of Streptavidin-R-Phycoerythrin (hereinafter, referred to as SA-PE) (manufactured by Prozyme)] was added thereto. This mixture was allowed to stand still in the shade of 25°C for 10 min and then was washed with 1× PBS-TP twice. Next, 75 µL of 1× PBS-TP was added, and the fluorescence of complexes of beads and SA-PE was measured by Luminex System (manufactured by Luminex) to detect the signal of the measurement target substance.

### (6) Result

For the poly A polymerase reaction with Target-1 (3'-end LNA) in the 100% mouse blood plasma matrix, the examination results of MnCh concentration are illustrated in Fig. 1 to Fig. 3.

Fig. 1 is a result of the background (BG) in which Target-1 is not included. In Example 1, when the MnCl₂ concentration was greater than 15 mM and 40 mM or less, the background was greatly suppressed. The BG value within this MnCl₂ concentration range became a value lower than that when MgCl₂ buffer attached to the kit in Comparative Example 1 was used.

Fig. 2 and Fig. 3 are graphs illustrating the results of the MFI and the SN ratio when the concentration of Target-1 is 1 fmol and 10 fmol. As can be seen in the SN ratio graph of Example 1, when the MnCl₂ concentration in the buffer was greater than 15 mM and 40 mM or less, the SN ratio was greatly increased, and the value of the SN ratio was hundreds of times higher than that in the general poly A polymerase reaction of Comparative Example 1 (when the buffer attached to the commercially available kit: MgCl₂ was used).

From the above, it was found that when Target-1 is allowed to react with the poly A polymerase, it is possible to suppress the background and to greatly increase the SN ratio by adding MnCl₂ to the buffer, and adjusting the concentration to an appropriate range.

### [Example 2] About dependence on target oligonucleotide concentration in PAP Mn buffer (100% mouse blood plasma matrix)

### (1) Poly A addition reaction

### (1-1) Preparation of sample

Target oligonucleotide (Target-1; SEQ ID No. 1) was adjusted with mouse blood plasma (Charles River, Japan) so that a final concentration became a concentration of 0, 10, 31.6, 100, 316, 1000, 3160, and 10000 amol.

### (1-2) Preparation of poly A polymerase reaction solution

### (i) Preparation of PAP Mix by commercially available kit (Comparative Example 2)

A reagent attached with an A-Plus^{™} Poly (A) Polymerase Tailing Kit (CELLSCRIPT, product number: C-PAP5104H) was used. Then, a total of 35 µL of PAP Mix was prepared by mixing 4 µL of 10× reaction buffer (attached to the kit; 0.5 M Tris-HCl (pH 8.0), 2.5 M NaCl, and 100 mM MgCl₂), 4 µL of 10 mM ATP (attached to the kit), 1 µL of poly (A) polymerase (attached to the kit), and 26 µL of Nuclease-Free Water. When the PAP Mix attached to the kit was used, the final concentration of MgCh in the poly A polymerase anti-solution was 10 mM.

### (ii) Preparation of PAP Mix of the present invention (Example 2)

In order to add poly A to the 3'-end of Target-1, a PAP reaction was carried out by using a self-prepared 5x PAP Mn buffer. The reaction solution was adjusted by using a PCR plate so as to obtain a final concentration of 1× PAP Mn buffer, and 1 mM ATP in a total volume of 40 µL. Further, 4 U of poly A polymerase was added. Regarding MnCl₂ concentration, a 5x PAP Mn buffer was prepared such that the concentration in a 1× PAP Mn buffer became 22.5 mM, and was used for the experiment.

### (1-3) Poly A addition reaction

35 µL of the PAP Mix and 5 µL of the Target-1 solution were added to a PCR plate and were mixed (40 µL in total). By using a thermal cycler, the reaction solution was kept warm at 37°C for 60 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) complementary to Target-1 was bound to MicroPlex^{™} Microspheres of Luminex (Region No.: 43, product number: LC10043-01), through NH₂ modification at the 3'-end (referred to as measurement target substance capturing beads).

### (2-2) Composition of 1^{st} hybridization reaction solution

Preparation was carried out to obtain a final concentration of 1.6x supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 65 µL obtained through mixing with the PAP reaction solution.

### (2-3) 1^{st} hybridization reaction

25 µL of the 1st hybridization reaction solution was added to the oligonucleotide solution obtained after the poly A addition reaction, and mixing was performed (65 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 1.6x supplement and 2.1 M TMAC, in 100 µL obtained through mixing with the 1st hybridization reaction solution. As a pair of self-aggregating probes, HCP-1 (SEQ ID No. 3), and HCP-2 (SEQ ID No. 4), in which the 5'-end was labeled with biotin, were used. HCP-1 and HCP-2 were added so that their respective final concentrations became 0.7 pmol/µL, and 0.56 pmol/µL, respectively.

### (3-2) Formation reaction of complex for detection (self-aggregating reaction/PALSAR reaction)

35 µL of the PALSAR reaction solution was added to 65 µL of the reaction solution obtained after the 1st hybridization step to make a total of 100 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1×PBS-TP [1×PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min and then was washed with 1× PBS-TP twice. Next, 75 µL of 1× PBS-TP was added, and the fluorescence from the complex of beads and SA-PE was measured by Luminex System (manufactured by Luminex) to detect the signal of the measurement target substance.

### (6) Test result

### (6-1) Concentration dependence

Figs. 4 to 6 illustrate results of the Target-1 concentration dependence in the PAP Mn buffer, regarding the 100% mouse blood plasma matrix.

Fig. 4 illustrates the linearity of the Target-1 concentration in the PAP Mn buffer (Example 2), regarding the 100% mouse blood plasma matrix. In a range of 0 to 1000 amol of the Target-1 concentration, the R² value of 0.9943 was obtained. Thus, a good linearity was obtained, which is not inferior even compared to the straight line (Fig. 5) illustrating the target concentration dependence in the case where Mg buffer attached to the kit in Comparative Example 2 was used.

### (6-2) SN ratio

Fig. 6 illustrates a comparison in the SN ratio between the Mn buffer (Example 2) and the Mg buffer (Comparative Example 2) at each Target concentration. It is illustrated that the SN ratio of the Mn buffer (Example 2) was greatly increased as compared to the Mg buffer (Comparative Example 2) at any Target-1 concentration. Further, it is illustrated that in the Mg buffer (Comparative Example 2), at the Target concentration of 10 amol, the SN ratio was 2, and then in the Mg buffer, the sensitivity was not obtained at a target concentration of 10 amol or less. Meanwhile, it is illustrated that in the Mn buffer (Example 2), at the Target-1 concentration of 10 amol, the SN ratio was 9, and then the sensitivity was obtained even at 10 amol or less.

From the above, it was found that the Target concentration dependence is good in the Mn buffer, and quantitativeness is achieved.

Further, it was found that it is possible to increase the detection sensitivity by using the Mn buffer in the present invention.

### [Example 3] About dependence on target oligonucleotide concentration in PAP 17.5 mM Mn buffer (100% mouse blood plasma matrix)

### (1) Poly A addition reaction

### (1-1) Preparation of sample

Target oligonucleotide (Target-1; SEQ ID No. 1) was adjusted with mouse blood plasma (Charles River, Japan) so that a final concentration became a concentration of 0, 10, 31.6, 100, 316, 1000, and 3160 amol.

### (1-2) Preparation of poly A polymerase reaction solution

### (i) Preparation of PAP Mix by commercially available kit (Comparative Example 3)

A reagent attached with an A-Plus^{™} Poly (A) Polymerase Tailing Kit (CELLSCRIPT, product number: C-PAP5104H) was used. Then, a total of 35 µL of PAP Mix was prepared by mixing 4 µL of 10× reaction buffer (attached to the kit; 0.5 M Tris-HCl (pH 8.0), 2.5 M NaCl, and 100 mM MgCl₂), 4 µL of 10 mM ATP (attached to the kit), 1 µL of poly (A) polymerase (attached to the kit), and 26 µL of Nuclease-Free Water. When the PAP Mix attached to the kit was used, the final concentration of MgCl₂ in the poly A polymerase anti-solution was 10 mM.

### (ii) Preparation of PAP Mix of the present invention (Example 3)

In order to add poly A to the 3'-end of Target-1, a PAP reaction was carried out by using a self-prepared 5x PAP Mn buffer. The reaction solution was adjusted by using a PCR plate so as to obtain a final concentration of 1×PAP Mn buffer, and 1 mM ATP, in a total volume of 40 µL. Further, 4 U of poly A polymerase was added. Regarding MnCl₂ concentration, a 5xPAP Mn buffer was prepared such that the concentration in a 1× PAP Mn buffer became 17.5 mM, and then was used for the experiment.

### (1-3) Poly A addition reaction

35 µL of the PAP Mix and 5 µL of the Target-1 solution were added to a PCR plate and were mixed (40 µL in total). By using a thermal cycler, the reaction solution was kept warm at 37°C for 60 min, and then was thermally treated at 65°C for 10 min, and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) complementary to Target-1 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 0.8x supplement (10x supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 65 µL obtained through mixing with the PAP reaction solution.

### (2-3) 1st hybridization reaction

25 µL of the 1st hybridization reaction solution was added to the oligonucleotide solution obtained after the poly A addition reaction, and mixing was performed (65 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 0.8x supplement and 1.6 M TMAC, in 100 µL obtained through mixing with the 1st hybridization reaction solution. As a pair of self-aggregating probes, HCP-1 (SEQ ID No. 3), and HCP-2 (SEQ ID No. 4), in which the 5'-end was labeled with biotin, were used. HCP-1 and HCP-2 were added so that their respective final concentrations became 0.35 pmol/µL, and 0.245 pmol/µL, respectively.

### (3-2) Formation reaction of complex for detection (self-aggregating reaction/PALSAR reaction)

35 µL of the PALSAR reaction solution was added to 65 µL of the reaction solution obtained after the 1st hybridization step to make a total of 100 µL. This was subjected to the reaction at 42°C for 1 h, and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1×PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Test Result

### (6-1) Concentration dependence

Fig. 9 and Fig. 10 illustrate results of the Target-1 concentration dependence in the PAP 17.5 mM Mn buffer, regarding the 100% mouse blood plasma matrix.

Fig. 9 illustrates the linearity of the Target-1 concentration in the PAP 17.5 mM Mn buffer (Example 3), regarding the 100% mouse blood plasma matrix. In a range of 0 to 1000 amol of the Target-1 concentration, the R² value of 0.9976 was obtained. Thus, a good linearity was obtained.

### (6-2) SN ratio

Fig. 10 illustrates a comparison in the SN ratio between the 17.5 mM Mn buffer (Example 3) and the Mg buffer (Comparative Example 3) at each Target-1 concentration. It is illustrated that the SN ratio of the Mn buffer (Example 3) was greatly increased as compared to the Mg buffer (Comparative Example 3) at any Target-1 concentration. Further, it is illustrated that in the Mg buffer (Comparative Example 3), at the Target-1 concentration of 10 amol, the SN ratio was 2, and then in the Mg buffer, the sensitivity was not obtained at a Target-1 concentration of 10 amol or less. Meanwhile, it is illustrated that in the Mn buffer (Example 3), at the Target-1 concentration of 10 amol, the SN ratio was 8, and then the sensitivity was obtained even at 10 amol or less.

From the above, it was found that the Target-1 concentration dependence is good in the 17.5 mM Mn buffer, and quantitativeness is achieved. Further, it was found that it is possible to increase the detection sensitivity by using the 17.5 mM Mn buffer in the present invention.

### [Example 4-1] About MnCl₂ concentration during poly A addition reaction by poly A polymerase (50% mouse blood plasma matrix)

### (1) Poly A addition reaction

### (1-1) Preparation of sample

Target-2 (target oligonucleotide) having a sequence illustrated below was adjusted with 50% mouse blood plasma matrix so that a final concentration became a concentration of 0, and 0.1 fmol. As the 50% mouse blood plasma matrix, mouse blood plasma (Charles River, Japan) diluted two times with RNase-Free Water was used.
<Sequence of target-2>
   T(L)^G(L)^A(L)^g^c^t^g^a^c^t^t^g^a^T(L)^G(L)^T(L) (SEQ ID No. 5 regarding a base sequence part)
"(L)" indicates "LNA."
"^" indicates "S-modified (phosphorothioate)."

### (1-2) Preparation of poly A polymerase reaction solution

### (i) Preparation of PAP Mix by commercially available kit (Comparative Example 4-1)

A reagent attached with a Poly (A) Tailing Kit (Invitrogen, product number: AM1350) was used to prepare a total of 10 µL of PAP Mix. A total of 10 µL of PAP Mix was prepared by mixing 4 µL of 5x E-PAP buffer (attached to the kit), 2 µL of 10 mM ATP solution (attached to the kit), 0.4 µL of E-PAP (attached to the kit), and 3.6 µL of Nuclease-Free Water.

### (ii) Preparation of PAP Mix of the present invention (Example 4-1)

In order to add poly A to the 3'-end of Target-2, a PAP reaction was carried out by using a self-prepared 5x PAP Mn buffer. The reaction solution was adjusted by using a PCR plate so as to obtain a final concentration of 1× PAP Mn buffer, and 1 mM ATP in a total volume of 20 µL. Further, 0.8 U of poly A polymerase was added. Regarding MnCl₂ concentration, a 5xPAP Mn buffer was prepared such that the concentration in a 1× PAP Mn buffer became 5, 10, 15, 17.5, 20, 22.5, 25, 30, 35, and 40 mM, and then was used for the experiment.

### (1-3) Poly A addition reaction

10 µL of the PAP Mix and 10 µL of the target-2 solution were added to a PCR plate and were mixed (20 µL in total). By using a thermal cycler (Mastercycler nexus GSX1 manufactured by Eppendorf), the reaction solution was kept warm at 37°C for 60 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) partially complementary to Target-2 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 0.8× supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 35 µL obtained through mixing with the PAP reaction solution.

### (2-3) 1st hybridization reaction

15 µL of the 1st hybridization reaction solution was added to the oligonucleotide solution obtained after the poly (A) addition reaction, and mixing was performed (35 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 0.8x supplement and 1.6 M TMAC, in 50 µL obtained through mixing with the 1st hybridization reaction solution. As a pair of self-aggregating probes, HCP-1 (SEQ ID No. 3) and HCP-2 (SEQ ID No. 4), in which the 5'-end was labeled with biotin, were used. HCP-1 and HCP-2 were added so that their respective final concentrations became 0.7 pmol/µL, and 0.49 pmol/µL, respectively.

### (3-2) Formation reaction of complex for detection (self-aggregating reaction/PALSAR reaction)

15 µL of the PALSAR reaction solution was added to 35 µL of the reaction solution obtained after the 1st hybridization step to make a total of 50 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

For the poly A polymerase reaction with Target-2 (3'-end LNA) in the 50% mouse blood plasma matrix, the examination results of MnCh concentration are illustrated in Fig. 11. This is a graph illustrating the results of the MFI and the SN ratio when the concentration of Target-2 is 0.1 fmol. As can be seen in the SN ratio graph of Example 4-1, when the MnCl₂ concentration in the buffer was 15 mM or more and 35 mM or less, the SN ratio was increased, and the value of the SN ratio was higher than that in the general poly A polymerase reaction of Comparative Example 4-1 (when the buffer attached to the commercially available kit: MgCl₂ was used).

From the above, it was found that even in the two-fold diluted blood plasma matrix, when Target-2 is allowed to react with the poly A polymerase, it is possible to suppress the background and to greatly increase the SN ratio by adding MnCl₂ to the buffer and adjusting the concentration to an appropriate range.

### [Example 4-2] About MnCl₂ concentration during poly A addition reaction by poly A polymerase (10% mouse blood plasma matrix)

### (1) Poly A addition reaction

### (1-1) Preparation of sample

Target oligonucleotide (Target-2; SEQ ID No. 5) was adjusted with 10% mouse blood plasma matrix so that a final concentration became a concentration of 0, and 0.1 fmol. As the 10% mouse blood plasma matrix, mouse blood plasma (Charles River, Japan) diluted ten times with RNase-Free Water was used.

### (1-2) Preparation of poly A polymerase reaction solution

### (i) Preparation of PAP Mix by commercially available kit (Comparative Example 4-2)

A reagent attached with a Poly (A) Tailing Kit (Invitrogen, product number: AM1350) was used to prepare a total of 10 µL of PAP Mix. A total of 10 µL of PAP Mix was prepared by mixing 4 µL of 5x E-PAP buffer (attached to the kit), 2 µL of 10 mM ATP solution (attached to the kit), 0.2 µL of E-PAP (attached to the kit), and 3.8 µL of Nuclease-Free Water.

### (ii) Preparation of PAP Mix of the present invention (Example 4-2)

In order to add poly A to the 3'-end of Target-2, a PAP reaction was carried out by using a self-prepared 5x PAP Mn buffer. The reaction solution was adjusted by using a PCR plate so as to obtain a final concentration of 1× PAP Mn buffer, and 1 mM ATP in a total volume of 20 µL. Further, 0.4 U of poly A polymerase was added. Regarding MnCl₂ concentration, a 5xPAP Mn buffer was prepared such that the concentration in a 1× PAP Mn buffer became 5, 10, 15, 17.5, 20, 22.5, 25, 30, and 35 mM, and then was used for the experiment.

### (1-3) Poly A addition reaction

10 µL of the PAP Mix and 10 µL of the target-2 solution were added to a PCR plate and were mixed (20 µL in total). By using a thermal cycler (Mastercycler nexus GSX1 manufactured by Eppendorf), the reaction solution was kept warm at 37°C for 30 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) partially complementary to Target-2 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 0.8× supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 35 µL obtained through mixing with the PAP reaction solution.

### (2-3) 1st hybridization reaction

15 µL of the 1st hybridization reaction solution was added to the oligonucleotide solution obtained after the poly (A) addition reaction, and mixing was performed (35 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 0.8x supplement and 1.6 M TMAC, in 50 µL obtained through mixing with the 1st hybridization reaction solution. As a pair of self-aggregating probes, HCP-1 (SEQ ID No. 3) and HCP-2 (SEQ ID No. 4), in which the 5'-end was labeled with biotin, were used. HCP-1 and HCP-2 were added so that their respective final concentrations became 0.7 pmol/µL, and 0.49 pmol/µL, respectively.

### (3-2) Formation reaction of complex for detection (self-aggregating reaction/PALSAR reaction)

15 µL of the PALSAR reaction solution was added to 35 µL of the reaction solution obtained after the 1st hybridization step to make a total of 50 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

For the poly A polymerase reaction with Target-2 (3'-end LNA) in the 10% mouse blood plasma matrix, the examination results of MnCh concentration are illustrated in Fig. 12. This is a graph illustrating the results of the MFI and the SN ratio when the concentration of Target-2 is 0.1 fmol. As can be seen in the SN ratio graph of Example 4-2, when the MnCl₂ concentration in the buffer was 10 mM or more and 25 mM or less, each SN ratio was increased, and the value of the SN ratio was higher than that in the general poly A polymerase reaction of Comparative Example 4-2 (when the buffer attached to the commercially available kit: MgCl₂ was used).

From the above, it was found that even in the ten-fold diluted blood plasma matrix, when Target-2 is allowed to react with the poly A polymerase, it is possible to suppress the background and to greatly increase the SN ratio by adding MnCl₂ to the buffer and adjusting the concentration to an appropriate range.

### [Example 5] About MnCl₂ concentration during poly A addition reaction by poly A polymerase (water)

### (1) Poly A addition reaction

### (1-1) Preparation of sample

Target oligonucleotide (Target-2; SEQ ID No. 5) was adjusted with Nuclease-Free Water so that a final concentration became a concentration of 0, and 0.1 fmol.

### (1-2) Preparation of poly A polymerase reaction solution

### (i) Preparation of PAP Mix by commercially available kit (Comparative Example 5)

A reagent attached with an A-Plus^{™} Poly(A) Polymerase Tailing Kit (CELLSCRIPT, product number: C-PAP5104H) was used. Then, a total of 10 µL of PAP Mix was prepared by mixing 2 µL of 10× reaction buffer (attached to the kit; 0.5 M Tris-HCl (pH 8.0), 2.5 M NaCl, and 100 mM MgCl₂), 2 µL of 10 mM ATP (attached to the kit), 0.25 µL of poly(A) polymerase (attached to the kit), and 5.75 µL of Nuclease-Free Water. When the PAP Mix attached to the kit was used, the final concentration of MgCh in the poly A polymerase anti-solution was 10 mM.

### (ii) Preparation of PAP Mix of the present invention (Example 5)

In order to add poly A to the 3'-end of Target-2, a PAP reaction was carried out by using a self-prepared 5x PAP Mn buffer. The reaction solution was adjusted by using a PCR plate so as to obtain a final concentration of 1× PAP Mn buffer, and 1 mM ATP, in a total volume of 10 µL. Further, 1 U of poly A polymerase was added. Regarding MnCl₂ concentration, a 5xPAP Mn buffer was prepared such that the concentration in a 1× PAP Mn buffer became 2.5, 5, 7.5, 15, 17.5, and 20 mM, and then was used for the experiment.

### (1-3) Poly A addition reaction

10 µL of the PAP Mix, and 10 µL of the target-2 solution were added to a PCR plate and were mixed (20 µL in total). By using a thermal cycler (Mastercycler nexus GSX1 manufactured by Eppendorf), the reaction solution was kept warm at 37°C for 5 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) partially complementary to Target-2 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 1.6x supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 35 µL obtained through mixing with the PAP reaction solution.

### (2-3) 1st hybridization reaction

15 µL of the 1st hybridization reaction solution was added to the oligonucleotide solution obtained after the poly (A) addition reaction, and mixing was performed (35 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 1.6x supplement and 1.6 M TMAC, in 50 µL obtained through mixing with the 1st hybridization reaction solution. As a pair of self-aggregating probes, HCP-1 (SEQ ID No. 3), and HCP-2 (SEQ ID No. 4), in which the 5'-end was labeled with biotin, were used. HCP-1 and HCP-2 were added so that their respective final concentrations became 0.7 pmol/µL, and 0.49 pmol/µL, respectively.

### (3-2) Formation reaction of complex for detection (self-aggregating reaction/PALSAR reaction)

15 µL of the PALSAR reaction solution was added to 35 µL of the reaction solution obtained after the 1st hybridization step to make a total of 50 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

For the poly A polymerase reaction with Target-2 (3'-end LNA) in water, the examination results of MnCh concentration are illustrated in Fig. 13. This is a graph illustrating the results of the MFI and the SN ratio when the concentration of Target-2 is 0.1 fmol. As can be seen in the SN ratio graph of Example 5, when the MnCl₂ concentration in the buffer was 5 mM or more and 15 mM or less, the SN ratio was increased, and the value of the SN ratio was several times higher than that in the general poly A polymerase reaction of Comparative Example 5 (when the buffer attached to the commercially available kit: MgCl₂ was used).

From the above, it was found that in water (buffer), when Target-2 is allowed to react with the poly A polymerase, it is possible to suppress the background and to increase the SN ratio by adding MnCl₂ to the buffer and adjusting the concentration to an appropriate range.

### [Example 6] About types of modification nucleic acid at 3'-end of target oligonucleotide in PAP Mn buffer (100% mouse blood plasma matrix)

### (1) Poly A addition reaction

### (1-1) Preparation of sample

Targets-3, 4, 5, and 6 (target oligonucleotide) having sequences illustrated below were adjusted with mouse blood plasma (Charles River, Japan) so that a final concentration became a concentration of 0 and 1 fmol.
<Sequence of target-3>
   T(L)^G(L)^A(L)^g^c^t^g^a^c^t^t^g^a^T(L)^G(L)^5(E) (SEQ ID No. 1 regarding a base sequence part)
<Sequence of target-4>
   T(L)^G(L)^A(L)^g^c^t^g^a^c^t^t^g^a^T(L)^G(L)^5(m) (SEQ ID No. 1 regarding a base sequence part)
<Sequence of target-5>
   T(L)^G(L)^A(L)^g^c^t^g^a^c^t^t^g^a^T(L)^G(L)^5(M) (SEQ ID No. 1 regarding a base sequence part)
<Sequence of target-6>
   T(L)^G(L)^A(L)^g^c^t^g^a^c^t^t^g^a^T(L)^G(L)^c (SEQ ID No. 1 regarding a base sequence part)
"(E)" indicates "BNA-NC(N-Me)."
"(m)" indicates "MOE."
"(M)" indicates "OMe."
"^" indicates "S-modified (phosphorothioate)."

### (1-2) Preparation of poly A polymerase reaction solution

### (i) Preparation of PAP Mix by commercially available kit (Comparative Example 6)

A reagent attached with a Poly (A) Tailing Kit (Invitrogen, product number: AM1350) was used. Then, a total of 35 µL of PAP Mix was prepared by mixing 8 µL of 5x E-PAP buffer (attached to the kit), 4 µL of 10 mM ATP solution (attached to the kit), 1.6 µL of E-PAP (attached to the kit), and 21.4 µL of Nuclease-Free Water.

### (ii) Preparation of PAP Mix of the present invention (Example 6)

In order to add poly A to the 3'-end of Targets-3, 4, 5, and 6, a PAP reaction was carried out by using a self-prepared 5x PAP Mn buffer. The reaction solution was adjusted by using a PCR plate so as to obtain a final concentration of 1× PAP Mn buffer and 1 mM ATP, in a total volume of 40 µL. Further, 3.2 U of poly A polymerase was added. Regarding MnCl₂ concentration, a 5x PAP Mn buffer was prepared such that the concentration in a 1× PAP Mn buffer became 22.5 mM, and then was used for the experiment.

### (1-3) Poly A addition reaction

35 µL of the PAP Mix and 5 µL of each Target solution were added to a PCR plate and were mixed (40 µL in total). By using a thermal cycler, the reaction solution was kept warm at 37°C for 60 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) complementary to Targets-3, 4, 5, and 6 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 1.0× supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 65 µL obtained through mixing with the PAP reaction solution.

### (2-3) 1st hybridization reaction

25 µL of the 1st hybridization reaction solution was added to the oligonucleotide solution obtained after the poly A addition reaction, and mixing was performed (65 µL in total). The mixed solution was kept warm at 42°C for 1 h, and then was kept at 4°C.

### (3) Formation reaction of complex for detection (self-aggregating reaction/PALSAR reaction)

(3-1) Preparation of PALSAR reaction solution Addition was carried out to obtain a final concentration of 1.0× supplement and 1.6 M TMAC, in 100 µL obtained through mixing with the 1st hybridization reaction solution. As a pair of self-aggregating probes, HCP-1 (SEQ ID No. 3), and HCP-2(SEQ ID No. 4), in which the 5'-end was labeled with biotin, were used. HCP-1 and HCP-2 were added so that their respective final concentrations became 0.35 pmol/µL, and 0.245 pmol/µL, respectively.

### (3-2) Formation reaction of complex for detection (self-aggregating reaction/PALSAR reaction)

35 µL of the PALSAR reaction solution was added to 65 µL of the reaction solution obtained after the hybridization step to make a total of 100 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

Fig. 14 illustrates results for signals of Targets-3 (3'-end BNA-NC(N-Me)), 4 (3'-end MOE), 5 (3'-end OMe), and 6 (3'-end DNA) in the 100% mouse blood plasma matrix. This is a graph illustrating SN ratios in the Mn buffer (Example 6) and the Mg buffer (Comparative Example 6), for each Target. It is illustrated that the SN ratio of the Mn buffer (Example 6) was greatly increased as compared to the Mg buffer (Comparative Example 6), for all Targets.

From the above, it was found that detection may be carried out with a high sensitivity by using the Mn buffer in the present invention, for any type of modification nucleic acid at the 3'-end. Further, it was found that even when the nucleic acid base at the 3'-end is DNA, detection may be carried out with a high sensitivity by using the Mn buffer.

### [Example 7-1] About MnCl₂ concentration during poly A addition reaction by poly A polymerase (2% mouse brain (20 mg/ml) matrix)

### (1) Poly A addition reaction

### (1-1) Adjustment of mouse brain matrix

A solution obtained by mixing Direct PCR Lysis Reagent (VIAGEN biotec, product number: 102-T) with Proteinase K solution (Kanto Chemical Co., Inc., product number: 9034) at a ratio of 100:1 was added to a mouse brain sample (Charles River, Japan, product number: P00041) to make 200 mg/mL (20% brain matrix). The mouse brain was crushed in the solution by using Power Masher II (Nippi, product number: 891300), and then was kept warm at 55°C, at 800 rpm for 60 min. This was further thermally treated at 85°C, at 800 rpm for 45 min, and then after centrifugation at 25°C, at 1600×g for 15 min, the supernatant was collected. The collected supernatant was diluted ten times with RNase-Free Water.

### (1-2) Preparation of sample

Target oligonucleotide (Target-2; SEQ ID No. 5) was adjusted with 2% mouse brain matrix so that a final concentration became a concentration of 0, and 0.1 fmol.

### (1-3) Preparation of poly A polymerase reaction solution

### (i) Preparation of PAP Mix by commercially available kit (Comparative Example 7-1)

A reagent attached with a Poly (A) Tailing Kit (Invitrogen, product number: AM1350) was used. Then, a total of 10 µL of PAP Mix was prepared by mixing 4 µL of 5x E-PAP buffer (attached to the kit), 2 µL of 10 mM ATP (attached to the kit), 0.4 µL of E-PAP and 3.6 µL of Nuclease-Free Water.

### (ii) Preparation of PAP Mix of the present invention (Example 7-1)

In order to add poly A to the 3'-end of Target-2, a PAP reaction was carried out by using a self-prepared 5x PAP Mn buffer. The reaction solution was adjusted by using a PCR plate so as to obtain a final concentration of 1× PAP Mn buffer, and 1 mM ATP in a total volume of 10 µL. Further, 0.8 U of poly A polymerase was added. Regarding MnCl₂ concentration, a 5x PAP Mn buffer was prepared such that the concentration in a 1× PAP Mn buffer became 5, 7.5, 10, 12.5, 15, 17.5, and 20 mM, and then was used for the experiment.

### (1-4) Poly A addition reaction

10 µL of the PAP Mix and 10 µL of the target-2 solution were added to a PCR plate and were mixed (20 µL in total). By using a thermal cycler (Mastercycler nexus GSX1 manufactured by Eppendorf), the reaction solution was kept warm at 37°C for 30 min, and then was thermally treated at 65°C for 10 min, and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) partially complementary to Target-2 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 1.6x supplement (10× supplement; [500 mM Tris-HCl (pH 8.0), 40 mM EDTA (pH 8.0), 8% N-sodium lauroyl sarcosinate]), 1.1 M TMAC, and 500 measurement target substance capturing beads prepared in the (2-1), in 35 µL obtained through mixing with the PAP reaction solution.

### (2-3) 1st hybridization reaction

15 µL of the 1st hybridization reaction solution was added to the oligonucleotide solution obtained after the poly (A) addition reaction, and mixing was performed (35 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 1.6x supplement and 1.6 M TMAC, in 50 µL obtained through mixing with the 1st hybridization reaction solution. As a pair of self-aggregating probes, HCP-1 (SEQ ID No. 3), and HCP-2 (SEQ ID No. 4), in which the 5'-end was labeled with biotin, were used. HCP-1 and HCP-2 were added so that their respective final concentrations became 0.7 pmol/µL, and 0.49 pmol/µL, respectively.

### (3-2) Formation reaction of complex for detection (self-aggregating reaction/PALSAR reaction)

15 µL of the PALSAR reaction solution was added to 35 µL of the reaction solution obtained after the 1st hybridization step to make a total of 50 µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

Fig. 15 is a graph illustrating the results of the MFI and the SN ratio when the concentration of Target-2 (3'-end LNA) in the 2% mouse brain matrix (20 mg/mL) is 0.1 fmol. As can be seen in the SN ratio graph of Example 7-1, when the MnCl₂ concentration in the buffer was 10 to 17.5 M, the SN ratio was greatly increased, and then the value of the SN ratio was higher than that in the general poly A polymerase reaction of Comparative Example 7-1 (when the buffer attached to the commercially available kit: MgCl₂ was used). From the above, it was found that in the 2% mouse brain matrix, when Target-2 is allowed to react with poly A polymerase, it is possible to suppress the background and to greatly increase the SN ratio by adding MnCl₂ to the buffer and adjusting the concentration to an appropriate range.

### [Example 7-2] About MnCl₂ concentration during poly A addition reaction by poly A polymerase (4% mouse brain (40 mg/ml) matrix)

### (1) Poly A addition reaction

### (1-1) Adjustment of mouse brain matrix

A solution obtained by mixing Direct PCR Lysis Reagent (VIAGEN biotec, product number: 102-T) with Proteinase K solution (Kanto Chemical Co., Inc., product number: 9034) at a ratio of 100:1 was added to a mouse brain sample (Charles River, Japan, product number: P00041) to make 200 mg/mL (20% brain matrix). The mouse brain was crushed in the solution by using Power Masher II (Nippi, product number: 891300). Then, this was kept warm at 55°C, at 800 rpm for 60 min. This was further thermally treated at 85°C, at 800 rpm for 45 min, and then after centrifugation at 25°C, at 1600×g for 15 min, the supernatant was collected. The collected supernatant was diluted five times with RNase-Free Water.

### (1-2) Preparation of sample

Target oligonucleotide (Target-2; SEQ ID No. 5) was adjusted with 4% mouse brain matrix so that a final concentration became a concentration of 0, and 0.1 fmol.

### (1-3) Preparation of poly A polymerase reaction solution

### (i) Preparation of PAP Mix by commercially available kit (Comparative Example 7-2)

A reagent attached with a Poly (A) Tailing Kit (Invitrogen, product number: AM1350) was used. Then, a total of 10 µL of PAP Mix was prepared by mixing 4 µL of 5x E-PAP buffer (attached to the kit), 2 µL of 10 mM ATP (attached to the kit), 0.4 µL of E-PAP, and 3.6 µL of Nuclease-Free Water.

### (ii) Preparation of PAP Mix of the present invention (Example 7-2)

In order to add poly A to the 3'-end of Target-2, a PAP reaction was carried out by using a self-prepared 5x PAP Mn buffer. The reaction solution was adjusted by using a PCR plate so as to obtain a final concentration of 1× PAP Mn buffer, and 1 mM ATP in a total volume of 20 µL. Further, 0.8 U of poly A polymerase was added. Regarding MnCl₂ concentration, a 5x PAP Mn buffer was prepared such that the concentration in a 1× PAP Mn buffer became 5, 7.5, 10, 12.5, 15, 17.5, and 20 mM, and then was used for the experiment.

### (1-4) Poly A addition reaction

10 µL of the PAP Mix and 10 µL of the target-2 solution were added to a PCR plate and were mixed (20 µL in total). By using a thermal cycler (Mastercycler nexus GSX1 manufactured by Eppendorf), the reaction solution was kept warm at 37°C for 30 min, and then was thermally treated at 65°C for 10 min and was kept at 4°C.

### (2) Capturing of poly A-added oligonucleotide by capture probe (hybridization)

### (2-1) Preparation of capture probe

In the preparation, a nucleic acid probe CP-1 (SEQ ID No. 2) partially complementary to Target-2 was bound to MicroPlex^{™} Microspheres of Luminex (Region No.: 43, product number: LC10043-01) through NH₂ modification at the 3'-end (referred to as measurement target substance capturing beads).

### (2-2) Composition of 1st hybridization reaction solution

Preparation was carried out to obtain a final concentration of 1.6x supplement (10× supplement; [500 mM Tris-HCl (pH8.0), 40 mM EDTA (pH 8.0), 8% N-sodium lauroyl sarcosinate]), 1.1 M TMAC, and 500 measurement target substance capturing beads prepared in the (2-1), in 35 µL obtained through mixing with the PAP reaction solution.

### (2-3) 1st hybridization reaction

15 µL of the 1st hybridization reaction solution was added to the oligonucleotide solution obtained after the poly (A) addition reaction, and mixing was performed (35 µL in total). The mixed solution was kept warm at 42°C for 1 h, and then was kept at 4°C.

### (3) Formation reaction of complex for detection (self-aggregating reaction/PALSAR reaction)

### (3-1) Preparation of PALSAR reaction solution

Addition was carried out to obtain a final concentration of 1.6x supplement and 1.6 M TMAC, in 50 µL obtained through mixing with the 1st hybridization reaction solution. As a pair of self-aggregating probes, HCP-1 (SEQ ID No. 3), and HCP-2(SEQ ID No. 4), in which the 5'-end was labeled with biotin, were used. HCP-1, and HCP-2 were added so that their respective final concentrations became 0.7 pmol/µL, and 0.49 pmol/µL, respectively.

### (3-2) Formation reaction of complex for detection (self-aggregating reaction/PALSAR reaction)

15 µL of the PALSAR reaction solution was added to 35 µL of the reaction solution obtained after the 1st hybridization step to make a total of 50µL. This was subjected to the reaction at 42°C for 1 h and was kept at 4°C.

### (4) Separation step (washing step)

After the formation reaction of the complex for detection, through centrifugation at room temperature at 1000×g, for 1 min, beads were precipitated, and the supernatant was removed by snapping. Then, 1× PBS-TP [1× PBS (Nippon Gene), 0.02% Tween 20 (CALBIOCHEM), 1.5 ppm ProClin 300 (SIGMA)] was added, and through centrifugation at room temperature at 1000×g, for 1 min, beads were precipitated, and the supernatant was removed by snapping.

### (5) Detection step (fluorescence detection)

The reaction solution obtained after the complex forming reaction was washed with 1× PBS-TP once, and then 50 µL of a detection reagent [5 µg/mL of SA-PE (manufactured by Prozyme)] was added thereto. This mixture was allowed to stand still in the shade of 25°C for 10 min, and then was washed with 1× PBS-TP twice. Next, 75 µL of 1× PBS-TP was added, and the fluorescence from the complex of beads and SA-PE was measured by Luminex System (Manufactured by Luminex) to detect the signal of the measurement target substance.

### (6) Result

Fig. 16 is a graph illustrating the results of the MFI and the SN ratio when the concentration of Target-2 (3'-end LNA) in the 4% mouse brain (40 mg/ml) matrix is 0.1 fmol. As can be seen in the SN ratio graph of Example 7-2, when the MnCl₂ concentration in the buffer was 10 to 20 mM, the SN ratio was greatly increased, and then the value of the SN ratio was higher than that in the general poly A polymerase reaction of Comparative Example 7-2 (when the buffer attached to the commercially available kit: MgCl₂ was used).

From the above, it was found that in the 4% mouse brain matrix, when Target-2 is allowed to react with poly A polymerase, it is possible to suppress the background and to greatly increase the SN ratio by adding MnCl₂ to the buffer and adjusting the concentration to an appropriate range.

### INDUSTRIAL APPLICABILITY

According to the present invention, in the development of drugs such as artificial nucleic acid, and nucleic acid medicine, it is possible to accurately measure a drug concentration in the biological sample of an animal or a human to which a drug has been administered, for a pharmacokinetics and pharmacodynamics (PK/PD) screening test in an investigation stage, a safety test, a pharmacological test and a pharmacokinetic test in a non-clinical stage, and a clinical stage.

## Claims

1. A method of adding poly A to a 3'-end of a target oligonucleotide in a sample, in which the target oligonucleotide is an oligonucleotide or a DNA in which a nucleic acid base at the 3'-end is chemically modified, the method comprising:
(i) bringing poly A polymerase into contact with the target oligonucleotide in the sample in the presence of Mn²⁺.

2. A method of collecting a target oligonucleotide in a sample, in which the target oligonucleotide is an oligonucleotide or a DNA in which a nucleic acid base at a 3'-end is chemically modified, the method comprising:
(i) bringing poly A polymerase into contact with the target oligonucleotide in the sample in the presence of Mn²⁺, thereby adding poly A to the 3'-end of the target oligonucleotide;
(ii) bringing the sample into contact with a capture probe for capturing the target oligonucleotide to perform hybridization;
wherein the capture probe includes
(A) a nucleic acid probe, and
(B) a solid phase, an adapter, or a linker adjacent to a nucleotide at a 3'-end or 5'-end of the nucleic acid probe, and
the nucleic acid probe contains an entire sequence or a partial sequence of the target oligonucleotide; and
(iii) collecting a hybridization product included in the sample.

3. A method of detecting a target oligonucleotide in a sample, in which the target oligonucleotide is an oligonucleotide or a DNA in which a nucleic acid base at a 3'-end is chemically modified, the method comprising:
(i) bringing poly A polymerase into contact with the target oligonucleotide in the sample in the presence of Mn²⁺, thereby adding poly A to the 3'-end of the target oligonucleotide;
(ii) bringing the sample into contact with a capture probe for capturing the target oligonucleotide to perform hybridization;
wherein the capture probe includes
(A) a nucleic acid probe, and
(B) a solid phase, an adapter, or a linker adjacent to a nucleotide at a 3'-end or 5'-end of the nucleic acid probe, and
the nucleic acid probe contains an entire sequence or a partial sequence of the target oligonucleotide;
(iii) collecting a hybridization product included in the sample; and
(iv) detecting the collected hybridization product.

4. A method of detecting a target oligonucleotide in a sample, in which the target oligonucleotide is an oligonucleotide or a DNA in which a nucleic acid base at a 3'-end is chemically modified, the method comprising:
(i) bringing poly A polymerase into contact with the target oligonucleotide in the sample in the presence of Mn²⁺, thereby adding poly A to the 3'-end of the target oligonucleotide;
(ii) bringing the sample into contact with a capture probe for capturing the target oligonucleotide to perform hybridization;
wherein the capture probe includes
(A) a nucleic acid probe, and
(B) a solid phase, an adapter, or a linker adjacent to a nucleotide at a 3'-end or 5'-end of the nucleic acid probe, and
the nucleic acid probe contains an entire sequence or a partial sequence of the target oligonucleotide;
(iii) bringing a hybridization product included in the sample, into contact with a pair of self-aggregating signal amplification probes composed of first and second oligonucleotides, thereby forming a complex of the hybridization product and an oligonucleotide polymer obtained through self-aggregation of the first and second oligonucleotides; and
(iv) detecting the complex.

5. The detection method according to claim 4, wherein at least one of the first and second oligonucleotides contains a poly T sequence.

6. The detection method according to claim 4 or 5, wherein the (iv) forming of the complex includes coming in contact with an assist probe,
wherein the assist probe contains
a poly T sequence, and a sequence complementary to an entire sequence or a partial sequence of at least one of the first and second oligonucleotides.

7. The detection method according to any one of claims 4 to 6, wherein the first oligonucleotide contains at least a nucleic acid region X, a nucleic acid region Y, and a nucleic acid region Z or a nucleic acid region Z containing a poly T sequence in order from an 5'-end side, and the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.

8. The method according to any one of claims 1 to 7, wherein the sample is a blood-derived component, a brain-derived component, a buffer, or water, which contains the target oligonucleotide.

9. The method according to any one of claims 1 to 8, wherein the oligonucleotide in which the nucleic acid base at the 3'-end is chemically modified is LNA, BNA, phosphorothioate, morpholino oligo, boranophosphate, 2'-O-methylated RNA(2'-OMe), 2'-O-methoxyethylated RNA(2'-MOE), or 2'-F.

10. The method according to claim 9, wherein the oligonucleotide in which the nucleic acid base at the 3'-end is chemically modified is LNA, BNA, MOE, or OMe.

11. The method according to any one of claims 1 to 10, wherein the adding of the poly A is performed in a solution containing Mn²⁺ of 3 mM to 38 mM.

12. A poly A polymerase reaction solution composition kit comprising:
(1) poly A polymerase;
(2) a reaction buffer solution that contains Mn²⁺ such that a final concentration of Mn²⁺ is 3 mM to 38 mM in a poly A polymerase reaction solution; and
(3) ATP

13. A kit of detecting a target oligonucleotide in a sample, in which the target oligonucleotide is an oligonucleotide or a DNA in which a nucleic acid base at a 3'-end is chemically modified, the kit comprising:
(1) poly A polymerase
(2) a reaction buffer solution that contains Mn²⁺ such that a final concentration of Mn²⁺ is 3 mM to 38 mM in a poly A polymerase reaction solution;
(3) a capture probe for capturing the target oligonucleotide; and
(4) a pair of self-aggregating probes composed of first and second oligonucleotides.

14. The kit according to claim 12, wherein the first oligonucleotide contains at least a nucleic acid region X, a nucleic acid region Y, and a nucleic acid region Z or a nucleic acid region Z containing a poly T sequence in order from an 5'-end side, and
the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.

15. A pair of self-aggregating probes composed of first and second oligonucleotides, which is used for the detection method according to claims 4 to 7, wherein the first oligonucleotide contains at least a nucleic acid region X, a nucleic acid region Y, and a nucleic acid region Z or a nucleic acid region Z containing a poly T sequence in order from an 5'-end side, and
the second oligonucleotide contains at least a nucleic acid region X' complementary to the nucleic acid region X, a nucleic acid region Y' complementary to the nucleic acid region Y, and a nucleic acid region Z' complementary to the nucleic acid region Z or a nucleic acid region Z' containing a poly A sequence in order from an 5'-end side.
